(19) Europäisches Patentamt
European Patent Office
Office européen des brevets

(11) EP 2 177 606 A2

(12) DEMANDE DE BREVET EUROPEEN

(43) Date de publication:
**21.04.2010 Bulletin 2010/16**

(21) Numéro de dépôt: **09180233.0**

(22) Date de dépôt: **12.09.2002**

(51) Int Cl.:
***C12N 9/10*** (2006.01) ***C12N 15/54*** (2006.01)
***C12N 15/63*** (2006.01) ***C12N 1/21*** (2006.01)
***C12N 15/74*** (2006.01) ***C07K 16/12*** (2006.01)
***C12P 19/38*** (2006.01)

(84) Etats contractants désignés:
**AT BE BG CH CY CZ DE DK EE ES FI FR GB GR IE IT LI LU MC NL PT SE SK TR**

(30) Priorité: **14.09.2001 FR 0111911**

(62) Numéro(s) de document de la (des) demande(s) initiale(s) en application de l'article 76 CBE:
**02798763.5 / 1 427 819**

(71) Demandeur: **INSTITUT PASTEUR
75015 Paris (FR)**

(72) Inventeur: **La désignation de l'inventeur n'a pas encore été déposée**

(74) Mandataire: **Santarelli
14 Avenue de la Grande Armée
B.P. 237
75822 Paris Cedex 17 (FR)**

Remarques:
Cette demande a été déposée le 21-12-2009 comme demande divisionnaire de la demande mentionnée sous le code INID 62.

# (54) N-désoxyribosyltransferases de lactobacilles, séquences nucléotidiques correspondantes et leurs applications

(57) La présente invention concerne de nouveaux polypeptides et leurs fragments, isolés de Lactobacillus, ayant au moins une activité N-désoxyribosyltransférase, les polynucléotides, les polynucléotides codant et/ou d'expression incluant lesdits vecteurs et les anticorps spécifiques dirigés contre lesdits polypeptides. L'invention concerne également un procédé de synthèse enzymatique de désoxyribonucléosides.

EP 2 177 606 A2

## Description

La présente invention est relative au domaine de la biologie, et plus particulièrement de la production microbiologique d'analogues de bases. La présente invention concerne de nouveaux polypeptides et leurs fragments, isolés de *Lactobacillus*, ayant au moins une activité N-désoxyribosyltransférase, les polynucléotides codant lesdits polypeptides, les vecteurs de clonage et/ou d'expression incluant lesdits polynucléotides, les cellules transformées par lesdits vecteurs et les anticorps spécifiques dirigés contre lesdits polypeptides. L'invention concerne également un procédé de synthèse enzymatique de désoxyribonucléosides.

Les analogues de nucléosides dont la structure comporte des altérations du sucre ou de la base hétérocyclique, forment une famille de molécules actives dans le traitement de nombreuses infections bactériennes, virales, parasitaires et fongiques ainsi que dans la chimiothérapie antitumorale [Périgaud et coll, 1992]. Par ailleurs les propriétés insecticides et herbicides de certains antibiotiques nucléosidiques en font des agents potentiels dans le secteur de l'agrochimie et de l'environnement [Isono, 1988]. L'industrie emploie deux modes de production de ces analogues, la synthèse organique et la conversion biocatalytique (conversion enzymatique et conversion microbiologique), qui présentent avantages et inconvénients opposés. La synthèse organique permet d'accéder aux structures chimiques les plus variées mais nécessite des étapes multiples et coûteuses en réactifs et en solvants. Au contraire, les méthodes biocatalytiques permettent une production aisée en milieu aqueux mais limitée à un petit nombre de composés possibles du fait de la spécificité des enzymes, qui admettent une gamme limitée d'analogues à la place de leurs substrats physiologique. Les nucléosides phosphorylases et N-désoxyribosyltransférase, qui proviennent des voies de sauvegarde des purines et pyrimidines chez les bactéries, sont les enzymes les plus utilisées pour ces conversions enzymatiques (Krenisky et coll, 1981).

Il existe donc une nécessité urgente d'obtenir des enzymes de conversion de nucléosides et de leurs dérivés, ayant une activité enzymatique élargie afin de diversifier la production industrielle de ces composés. C'est le problème technique que se propose de résoudre les inventeurs de la présente invention.

La N-désoxyribosyltransférase de *Lactobacillus leichmannii* ainsi que celle de L. helveticus, partiellement purifiée ou non, se révèle être le meilleur donneur de groupement glycosyl et tolère un nombre important de variations structurales sur la base. Cette enzyme a été utilisée pour produire un certain nombre d'analogues parmi lesquels il convient de citer la 2-chloro,2'-désoxyadénosine (Carson et coll, 1984), les 2',3'-didésoxynucléosides des bases naturelles (Carson et Wasson, 1988) ou encore plusieurs pyrazolo(3,4-d)pyrimidine et triazolo(4,5-d)pyrimidine dérivés de la 2',3'-didésoxycytidine et de la base correspondante (Fischer et coll. 1990).

Dans le but de disposer d'enzymes recombinantes capables de traiter la plus large variété de substrats déviants soit par la base ou par le sucre, les inventeurs ont isolé des gènes codant pour une activité N-désoxyribosyltransférase de différentes souches de lactobacilles. Cette variété d'enzymes N-désoxyribosyltransférase permet d'augmenter les chances d'obtenir des enzymes à la spécificité élargie par des mutations dans les gènes sauvages ou par des chimères de ces gènes sauvages.

Deux classes de N-désoxyribosyltransférase ont été distinguées (Danzin et Cardinaud, 1976), la première (classe I) désigné ptd (pour purine transdésoxyribosylase) catalysant exclusivement l'échange de désoxyribose entre deux purine:

dR-Pur + Pur' <-> dR-Pur' + Pur

et la deuxième (classe II) désignés ntd (pour nucléoside transdésoxyribosylase), l'échange de désoxyribose entre une purine et une pyrimidine, entre deux pyrimidines ou entre deux purines:

dR-Pyr + Pur <-> dR-Pur + Pyr

dR-Pyr + Pyr' <-> dR-Pyr' + Pyr

dR-Pur + Pur' <-> dR-Pur' + Pur

Seuls deux gènes spécifiant des enzymes de classe II, désignés ntd, ont été rapportés à ce jour (Hück, 1997 ;

dbj|BAA92683.2| (AB039914)).

La présente invention a donc pour objet un polypeptide isolé ou purifié de *Lactobacillus* ayant au moins une activité N-désoxyribosyltransférase de séquence d'acides aminés choisi parmi les séquences SEQ ID N°2, SEQ ID N°4, SEQ ID N°6, SEQ ID N°8, SEQ ID N°10, SEQ ID N°12, SEQ ID N°14.

Selon un mode préféré de réalisation, le polypeptide selon l'invention est la N-désoxyribosyltransférase de SEQ ID N°2 (ou SEQ ID N°14) codée par le gène ntd Lh de *Lactobacillus helveticus*.

Selon un second mode préféré de réalisation, le polypeptide selon l'invention est la N-désoxyribosyltransférase de SEQ ID N°4 codée par le gène ptd Lh de *Lactobacillus helveticus*.

Selon un troisième mode préféré de réalisation, le polypeptide selon invention est la N-désoxyribosyltransférase de SEQ ID N°6 codée par le gène ntd Lf de *Lactobacillus fermentum*.

Selon un quatrième mode préféré de réalisation, le polypeptide selon l'invention est la N-désoxyribosyltransférase de SEQ ID N°8 codée par le gène ntd de *Lactobacillus crispatus*.

Selon un cinquième mode préféré de réalisation, le polypeptide selon l'invention est la N-désoxyribosyltransférase de SEQ ID N°10 codée par le gène ntd de *Lactobacillus amylovorus*.

Selon un sixième mode préféré de réalisation, le polypeptide selon l'invention est la N-désoxyribosyltransférase de SEQ ID N°12 codée par le gène ntd de *Lactobacillus acidophilus*.

Le polypeptide isolé selon l'invention se **caractérise en ce qu**'il comprend un polypeptide choisi parmi (a) un polypeptide de séquence SEQ ID N°2, SEQ ID N°4, SEQ ID N°6, SEQ ID N°8, SEQ ID N°10, SEQ ID N°12, SEQ ID N°14; (b) un polypeptide variant de polypeptide de séquences d'acides aminés défini en a) ; (c)un polypeptide homologue au polypeptide défini en (a) ou (b) et comportant au moins 80 % d'identité, de préférence 85 %, 87 %, 90 %, 95 % , 97 % 98 %, 99 % d'identité avec ledit polypeptide de a) ; (d) un fragment d'au moins 15 acides aminés consécutifs de préférence 17, 20, 23, 25, 30, 40, 50, 100, 250 amino-acides consécutifs d'un polypeptide défini en a), b) ou c) ; et (e) un fragment biologiquement actif d'un polypeptide défini en a), b) ou c).

Le polypeptide selon l'invention se **caractérise en ce qu**'il permet de satisfaire l'exigence en guanine de certaines souches bactériennes telles PAK6 qui est une souche d'*E. coli* dont les deux gènes de l'opéron *guaBA*, qui commandent la conversion de IMP en XMP puis en GMP, ainsi que ceux de l'opéron *deoCABD* qui commandent la dégradation des désoxynucléosides, ont été délétés. En effet, ces souches pour survivre ou croître nécessitent l'apport de désoxyguanosine (dR-G) dans le milieu de culture et la présence d'une activité N-désoxyribosyltransférase d'un polypeptide selon l'invention pour réaliser l'échange: dR-G + A <-> dR-A + G.

Dans la présente description, on utilisera le terme polypeptide pour désigner également une protéine ou un peptide.

On entendra par polypeptide variant l'ensemble des polypeptides mutés pouvant exister naturellement, en particulier chez l'être humain, et qui correspondent notamment à des troncatures, substitutions, délétions et/ou additions de résidus d'amino-acides.

Par polypeptide homologue, on entendra désigner les polypeptides présentant, par rapport aux désoxyribosyltransférases naturelles de *Lactobacillus* selon l'invention, certaines modifications comme en particulier une délétion, addition ou substitution d'au moins un acide aminé, une troncature, un allongement et/ou une fusion chimérique. Parmi les polypeptides homologues, on préfère ceux dont la séquence d'acides aminés présente au moins 80% d'identité, de préférence d'au moins 85%, 87%, 90%, 93%, 95%, 97%, 98%, 99% d'identité avec les séquences d'acides aminés des polypeptides selon l'invention. Dans le cas d'une substitution, un ou plusieurs acides aminés consécutifs ou non consécutifs, peuvent être remplacés par des acides aminés « équivalents ». L'expression acide aminé « équivalent » vise ici à désigner tout acide aminé susceptible d'être substitué à l'un des acides aminés de la structure de base sans cependant modifier les caractéristiques ou propriétés fonctionnelles essentielles, comme leurs activités biologiques (c'est-à-dire de désoxyribosyltransférase), des polypeptides correspondants telles que l'induction *in vivo* d'anticorps capables de reconnaître le polypeptide dont la séquence d'acides aminés est comprise dans la séquence d'acides aminés SEQ ID N°2, SEQ ID N°4, SEQ ID N°6, SEQ ID N°8, SEQ ID N°10, SEQ ID N°12, SEQ ID N°14, ou l'un de ses fragments. Ces acides aminés équivalents peuvent être déterminés soit en s'appuyant sur leur homologie de structure avec les acides aminés auxquels ils se substituent, soit sur les résultats des essais d'activité biologique croisée auxquels les différents polypeptide sont susceptibles de donner lieu. A titre d'exemple, on mentionnera les possibilités de substitutions susceptibles d'être effectuées sans qu'il en résulte une modification approfondie des activités biologiques des polypeptides modifiés correspondants, les remplacements, par exemple, de la leucine par la valine ou l'isoleucine, de l'acide aspartique par l'acide glutamique, de la glutamine par l'asparagine, de l'arginine par la lysine etc., les substitutions inverses étant naturellement envisageables dans les mêmes conditions.

Par fragment de polypeptide, on entend désigner un polypeptide comportant au minimum 15 acides aminés consécutifs, de préférence 17, 20, 23, 25, 30, 40, 50, 100, 250 amino-acides consécutifs. Les fragments de polypeptide selon l'invention obtenus par clivage dudit polypeptide par une enzyme protéolytique, par un réactif chimique, ou encore en plaçant ledit polypeptide dans un environnement très acide font également partie de l'invention.

Par fragment biologiquement actif, on entendra désigner en particulier un fragment de séquence d'acides aminés de polypeptide selon l'invention présentant au moins une des caractéristiques ou propriétés fonctionnelles du polypeptide selon l'invention, notamment en ce qu'il comporte une activité N-désoxyribosyltransférase. Le polypeptide variant, le polypeptide homologue ou le fragment de polypeptide selon l'invention possède au moins 10%, de préférence 20% , 30 , 40%, 50%, 60%, 70%, 80%, 90%, 95% de l'activité N-désoxyribosyltransférase.

Différents protocoles connus de l'homme de l'art ont été décrits pour introduire des mutations dans les polypeptides. Parmi ceux-ci il convient de citer à titre d'exemple la réaction de polymérisation en chaîne (PCR) en présence de manganèse (Cadwell *et al.*, 1992). Les mutations peuvent être introduites soit de manière aléatoire - dans ce cas l'étape de mutagenèse est suivie d'une étape de criblage du mutant d'intérêt - soit de manière criblée. Dans ce dernier cas, les mutations sont de préférence introduites au niveau du site catalytique des N-désoxyribosyltransférases selon l'invention.

De préférence un polypeptide selon l'invention est un polypeptide constitué de la séquence SEQ ID N°2, SEQ ID N°4, SEQ ID N°6, SEQ ID N°8, SEQ ID N°10, SEQ ID N°12, SEQ ID N°14 ou d'une séquence possédant au moins 80% d'identité, de préférence au moins 85%, 90%, 95%, 98% et 99% d'identité avec la SEQ ID N°2, SEQ ID N°4, SEQ ID N°6, SEQ ID N°8, SEQ ID N°10, SEQ ID N°12, SEQ ID N°14 après alignement optimal. Par polypeptide dont la séquence d'acides aminés présentant un pourcentage d'identité d'au moins 80%, de préférence d'au moins 85%, 90%, 95%, 98% et 99% après alignement optimal avec une séquence de référence, on entend désigner les polypeptides présentant certaines modifications par rapport au polypeptide de référence, comme en particulier une ou plusieurs délétions, troncations, un allongement, une fusion chimérique, et/ou une ou plusieurs substitutions.

Parmi les polypeptides dont la séquence d'acides aminés présente un pourcentage d'identité d'au moins 8.0%, de préférence d'au moins 85%, 90%, 95%, 98% et 99% après alignement optimal avec les séquences SEQ ID N° 2, SEQ ID N°4, SEQ ID N°6, SEQ ID N°8, SEQ ID N°10, SEQ ID N°12, SEQ ID N°14 ou avec l'un de leurs fragments selon l'invention, on préfère les polypeptides variants codés par les séquences peptidiques variantes telles que précédemment définies, en particulier les polypeptides dont la séquence d'acides aminés présente au moins une mutation correspondant notamment à une troncation, délétion, substitution et/ou addition d'au moins un résidu d'acide aminé par rapport aux séquences SEQ ID N°2, SEQ ID N°4, SEQ ID N°6, SEQ ID N°8, SEQ ID N°10, SEQ ID N°12, SEQ ID N°14 ou avec l'un de leurs fragments ; de manière plus préférée, les polypeptides variants présentant au moins une mutation qui diminue la spécificité du polypeptide selon l'invention pour son substrat, de sorte que les polypeptides variants selon l'invention sont capables de catalyser une plus large variété de substrat, afin d'obtenir une gamme plus étendue d'analogues de base.

L'invention concerne également un polynucléotide purifié ou isolé **caractérisé en ce qu**'il code pour un polypeptide tel que défini précédemment, et de préférence pour un polypeptide de séquence SEQ ID N°2, SEQ ID N°4, SEQ ID N°6, SEQ ID N°8, SEQ ID N°10, SEQ ID N°12, SEQ ID N°14. De manière préférée, le polynucléotide selon l'invention possède la séquence SEQ ID N°1, SEQ ID N°3, SEQ ID N°5, SEQ ID N°7, SEQ ID N°9, SEQ ID N°11, SEQ ID N°13.

Le polynucléotide isolé ou purifié selon l'invention se **caractérisé en ce qu**'il comprend un polynucléotide choisi parmi (a) SEQ ID N°1, SEQ ID N°3, SEQ ID N°5, SEQ ID N°7, SEQ ID N°9, SEQ ID N°11, SEQ ID N°13 ; (b) la séquence d'un fragment d'au moins 15 nucléotides consécutifs, de préférence d'au moins 18, 21, 24, 27, 30, 35, 40, 50, 75, 100, 150, 200 nucléotides consécutifs de la séquence SEQ ID N°1, SEQ ID N°3, SEQ ID N°5, SEQ ID N°7, SEQ ID N°9, SEQ ID N°11, SEQ ID N°13; (c) une séquence nucléique présentant un pourcentage d'identité d'au moins 70 %, de préférence d'au moins 75%, 80%, 85%, 90%, 95%, 98% et 99% après alignement optimal avec une séquence définie en a) ou b); (d) la séquence complémentaire ou la séquence d'ARN correspondant à une séquence telle que définie en a), b) ou c).

Le polynucléotide selon l'invention se caractérise également en ce que son expression dans les cellules hôtes, notamment les souches bactériennes telles PAK6, permet de satisfaire à l'exigence en guanine de la dite souche. La souche PAK6 a été déposée à la CNCM le 2 mai 2001 sous le N° 1-2664. La souche PAK6 correspond à la souche bactérienne d'*Escherichia coli* MG 1655 délétée de deux gènes guaA et guaB ainsi que des gènes déoC, déoA, déoB, déoD. La souche PAK6 (ΔguaBA::gm Δdeo-11) est auxotrophe pour la guanine en milieu minimal glucose.

Par acide nucléique, séquence nucléique ou d'acide nucléique, polynucléotide, oligonucléotide, séquence de polynucléotide, séquence nucléotidique, termes qui seront employés indifféremment dans la présente description, on entend désigner un enchaînement précis de nucléotides, modifiés ou non, permettant de définir un fragment ou une régions d'un acide nucléique, comportant ou non des nucléotides non naturels, et pouvant correspondre aussi bien à un ADN double brin, un ADN simple brin que des produits de transcription desdits ADN, et/ou un fragment d'ARN.

Il doit être compris que la présente invention ne concerne pas les séquences nucléotidiques dans leur environnement chromosomique naturel, c'est-à-dire à l'état naturel. Il s'agit de séquences qui ont été isolées et/ou purifiées, c'est-à-dire qu'elles ont été prélevées directement ou indirectement, par exemple par copie, leur environnement ayant été au moins partiellement modifié. On entend ainsi également désigner les acides nucléiques obtenus par synthèse chimique.

Par polynucléotide de séquence complémentaire, on entend désigner tout ADN dont les nucléotides sont complémentaires de ceux de la SEQ ID N°1, SEQ ID N°3, SEQ ID N°5, SEQ ID N°7, SEQ ID N°9, SEQ ID N°11, SEQ ID N°13 ou d'une partie de la SEQ ID N°1, SEQ ID N° 3, SEQ ID N° 5, SEQ ID N° 7, SEQ ID N° 9, SEQ ID N° 11, SEQ ID N°13 et dont l'orientation est inversée.

Par « pourcentage d'identité » entre deux séquences d'acides nucléiques ou d'acides aminés au sens de la présente invention, on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur. On entend désigner par "meilleur alignement" ou "alignement significatif", l'alignement pour lequel le pourcentage d'identité déterminé comme ci-après est le plus élevé. Les comparaisons de séquences entre deux séquences d'acides nucléiques ou d'acides aminés sont traditionnellement réalisées en comparant ces séquences après les avoir alignées de manière significative, ladite comparaison étant réalisée par segment ou par « fenêtre de comparaison » pour identifier et comparer les régions locales de similarité de séquence. L'alignement significatif des séquences pour la comparaison peut être réalisé, outre manuellement, au moyen de l'algorithme d'homologie locale de Smith et Waterman (1981), au moyen de l'algorithme d' homologie locale de Neddleman et Wunsch (1970), au moyen de la méthode de recherche de similarité de Pearson et Lipman (1988), au moyen de logiciels informatiques utilisant ces algorithme (GAP, BESTFIT, BLAST P, BLAST N, FASTA et TFASTA dans le Wisconsin Genetics Software Package, Genetics Computer Group, 575 Science Dr., Madison, WI). Afin d'obtenir l'alignement significatif, on utilise de préférence le programme BLAST, avec la matrice BLOSUM. 62. On peut également utiliser les matrices PAM ou PAM250.

Le pourcentage d'identité entre deux séquences d'acides nucléiques ou d'acides aminés est déterminé en comparant ces deux séquences alignées de manière significative, la séquence d'acides nucléiques ou d'acides aminés à comparer pouvant comprendre des additions ou des délétions par rapport à la séquence de référence pour un alignement significatif entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions comparées et en multipliant le résultat obtenu par 100 pour obtenir le pourcentage d'identité entre ces deux séquences.

Par séquences nucléiques présentant un pourcentage d'identité d'au moins 70%, de préférence d'au moins 75%, 80%, 85%, 90%, 95%, 98% et 99% après alignement significatif avec une séquence de référence, on entend désigner les séquences nucléiques présentant, par rapport à la séquence nucléique de référence, certaines modifications comme en particulier une délétion, une troncation, un allongement, une fusion chimérique, et/ou une substitution, notamment ponctuelle, et dont la séquence nucléique présente au moins 70%, de préférence au moins 75%, 80%, 85%, 90%, 95%, 98% et 99% d'identité après alignement significatif avec la séquence nucléique de préférence. Il s'agit de préférence de séquences dont les séquences complémentaires sont susceptibles de s'hybrider spécifiquement avec les séquences SEQ ID N°1, SEQ ID N°3, SEQ ID N°5, SEQ ID N°7, SEQ ID N°9, SEQ ID N°11, SEQ ID N°13 de l'invention. De préférence, les conditions d'hybridation spécifiques ou de forte stringence seront telles qu'elles assurent au moins 70%, de préférence d'au moins 75%, 80%, 85%, 90%, 95%, 98% et 99% d'identité après alignement significatif entre l'une des deux séquences et la séquence complémentaire de l'autre. Une hybridation dans des conditions de forte stringence signifie que les conditions de température et de force ionique sont choisies de telle manière qu'elles permettent le maintien de l'hybridation entre deux fragments d'ADN complémentaires. A titre illustratif, des conditions de forte stringence de l'étape d'hybridation aux fins de définir les fragments polynucléotidiques décrits ci-dessus, sont avantageusement les suivantes l'hybridation ADN-ADN ou ADN-ARN est réalisée en deux étapes : (1) préhybridation à 42°C pendant 3 heures en tampon phosphate (20 mM, pH 7,5) contenant 5 x SSC (1 x SSC correspond à une solution 0,15 M NaCl + 0,015 M citrate de sodium), 50 % de formamide, 7 % de sodium dodécyl sulfate (SDS), 10 x Denhardt's, 5 % de dextran sulfate et 1% d'ADN de sperme de saumon ; (2) hybridation proprement dite pendant 20 heures à une température dépendant de la taille de la sonde (c'est-à-dire : 42°C, pour une sonde de taille > 100 nucléotides) suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C pour une sonde de taille > 100 nucléotides. Les conditions d'hybridation de forte stringence décrites ci-dessus pour un polynucléotide de taille définie, peuvent être adaptées par l'homme du métier pour des oligonucléotides de taille plus grande ou plus petite, selon l'enseignement de Sambrook *et al.*, 1989.

Parmi les séquences nucléiques présentant un pourcentage d'identité d'au moins 70 %, de préférence d'au moins 75%, 80%, 85%, 90%, 95%, 98% et 99% après alignement significatif avec la séquence selon l'invention, on préfère également les séquences nucléiques variantes de SEQ ID N°1, SEQ ID N°3, SEQ ID N°5, SEQ ID N°7, SEQ ID N°9, SEQ ID N°11, SEQ ID N°13, ou de leurs fragments, c'est-à-dire l'ensemble des séquences nucléique correspondant à des variants alléliques, c'est-à-dire des variations individuelles des séquences SEQ ID N°1, SEQ ID N°3, SEQ ID N°5, SEQ ID N°7, SEQ ID N°9, SEQ ID N°11, SEQ ID N°13.

Plus particulièrement, l'invention concerne un acide nucléique purifié ou isolé selon la présente invention, **caractérisé en ce qu**'il comprend ou est constitué de l'une des séquences SEQ ID N°1, SEQ ID N°3, SEQ ID N°5, SEQ ID N°7, SEQ ID N°9, SEQ ID N°11, SEQ ID N°13 de leurs séquences complémentaires ou des séquences de l'ARN correspondant à SEQ ID N° 1, SEQ ID N°3, SEQ ID N°5, SEQ ID N°7, SEQ ID N°9, SEQ ID N°11, SEQ ID N°13. Les amorces ou sondes, **caractérisées en ce qu'**elles comprennent une séquence d'un acide nucléique selon l'invention, font également partie de l'invention. Ainsi, les amorces où les sondes selon l'invention sont utiles pour la détection, l'identification, le dosage ou l'amplification de séquence d'acide nucléique. En particulier, ils peuvent permettre de mettre en évidence ou de discriminer les séquences nucléiques variantes, ou d'identifier la séquence génomique de nouveaux gènes eucaryotes ou procaryotes, bactériens notamment, et plus précisément de bactéries *Lactobacillus,* codant pour une N-désoxyribosyltransférase, en utilisant notamment une méthode d'amplification telle que la méthode PCR, ou une méthode apparentée. Selon l'invention, les polynucléotides pouvant être utilisés comme sonde ou comme amorce dans des procédés de détection, d'identification, de dosage ou d'amplification de séquence nucléique, présentent une taille minimale de 10 bases, de préférence d'au moins 15, 18, 20, 25, 30, 40, 50 bases. Selon un mode de réalisation, les amorces selon l'invention sont choisies parmi les séquences SEQ ID N° 15 et SEQ ID N° 16.

Les polynucléotide selon l'invention peuvent ainsi être utilisés comme amorce et/ou sonde dans des procédés mettant en oeuvre notamment la technique de PCR (amplification en chaîne par polymérase) (Rolfs *et al.,* 1991). Cette technique nécessite le choix de paires d'amorces oligonucléotidiques encadrant le fragment qui doit être amplifié. On peut, par exemple, se référer à la technique décrite dans le brevet américain U.S. N° 4 683 202. Les fragments amplifiés peuvent être identifiés, par exemple après une électrophorèse en gel d'agarose ou de polyacrylamide, ou après une technique chromatographique comme la filtration sur gel ou la chromatographie échangeuse d'ions, puis séquences. La spécificité de l'amplification peut être contrôlée en utilisant comme amorces les séquences nucléotidiques de polynucléotides de l'invention et comme matrices, des plasmides contenant ces séquences ou encore les produits d'amplification dérivés. Les fragments nucléotidiques amplifiés peuvent être utilisés comme réactifs dans des réactions d'hybridation afin de mettre en évidence la présence, dans un échantillon biologique, d'un acide nucléique cible de séquence complémentaire à celle desdits fragments nucléotidiques amplifiés. L'invention vise également les acides nucléiques susceptibles d'être obtenus par amplification à l'aide d'amorces selon l'invention.

D'autres techniques d'amplification de l'acide nucléique cible peuvent être avantageusement employées comme alternative à la PCR (PCR-like) à l'aide de couple d'amorces de séquences nucléotidiques selon l'invention. Par PCR-like on entend désigner toutes les méthodes mettant en oeuvre des reproductions directes ou indirectes des séquences d'acides nucléiques, ou bien dans lesquelles les système de marquage ont été amplifiés, ces techniques sont bien entendu connus. En général il s'agit de l'amplification de l'ADN par une polymérase ; lorsque l'échantillon d'origine est un ARN il convient préalablement d'effectuer une transcription reverse. Il existe actuellement de très nombreux procédés permettant cette amplification, comme par exemple la technique SDA (Strand Displacement Amplification) ou technique d'amplification à déplacement de brin (Walker *et al.,* 1992), la technique TAS (Transcription-based Amplification System) décrite par Kwoh *et al.* (1989), la technique 3SR (Self-Sustained Sequence Replication) décrite par Guatelli *et al.* (1990), la technique NASBA (Nucleic Acid Sequence Based Amplification) décrite par Kievitis *et al.* (1991), la technique TMA (Transcription Mediated Amplification), la technique LCR (Ligase Chain Rection) décrite par Landegren *et al.* (1988), la technique de RCR (Repair Chain Reaction) décrite par Segev (1992), la technique CPR (Cycling Probe Reaction) décrite par Duck *et al.* (1990), la technique d'amplification à la Q-β-réplicase décrite par Miele *et al.* (1983). Certaines de ces techniques ont depuis été perfectionnées.

Dans le cas où le polynucléotide cible à détecter est un ARNm, on utilise avantageusement, préalablement à la mise en oeuvre d'une réaction d'amplification à l'aide des amorces selon l'invention ou à la mise en oeuvre d'un procédé de détection à l'aide des sondes de l'invention, une enzyme de type transcriptase inverse afin d'obtenir un ADNc à partir de l'ARNm contenu dans l'échantillon biologique. L'ADNc obtenu servira alors de cible pour les amorces ou les sondes mises en *oeuvre* dans le procédé d'amplification ou de détection selon l'invention.

La technique d'hybridation de sondes peut être réalisée de manières diverses (Matthews *et al*., 1988). La méthode la plus générale consiste à immobiliser l'acide nucléique extrait des cellules de différents tissus ou de cellules en culture sur un support (tels que la nitrocellulose, le nylon, le polystyrène) pour réaliser par exemple des puces à ADN, puis à incuber, dans des conditions bien définies, l'acide nucléique cible immobilisé avec la sonde. Après l'hybridation, l'excès de sonde est éliminé et les molécules hybrides formées sont détectées par la méthode appropriée (mesure de la radioactivité, de la fluorescence ou de l'activité enzymatique liée à la sonde).

Selon un autre mode de mise en oeuvre des sondes nucléiques selon l'invention, ces dernières peuvent être utilisées comme sondes de capture. Dans ce cas, une sonde, dite « sonde de capture », est immobilisée sur un support et sert à capturer par hybridation spécifique l'acide nucléique cible obtenu à partir de l'échantillon biologique à tester et l'acide nucléique cible est ensuite détecté grâce à une seconde sonde, dite « sonde de détection », marquée par un élément facilement détectable.

Parmi les fragments d'acides nucléiques intéressants, il convient par ailleurs de citer en particulier les oligonucléotides anti-sens, c'est-à-dire dont la structure assure, par hybridation avec la séquence cible, une inhibition de l'expression du produit correspondant. Il faut également citer les oligonucléotides sens qui, par interaction avec des protéines impliquées dans la régulation de l'expression du produit correspondant, induiront soit une inhibition, soit une activation de cette expression. Les oligonucléotides selon l'invention présente une taille minimale de 9 bases, de préférence d'au moins 10, 12, 15, 17, 20, 25, 30, 40, 50 bases.

Les sondes, amorces et oligonucléotides selon l'invention peuvent être marqués directement ou indirectement par un composé radioactif ou non radioactif, par des méthodes bien connues de l'homme du métier, afin d'obtenir un signal détectable et/ou quantifiable. Les séquences de polynucléotides selon l'invention non marquées peuvent être utilisées directement comme sonde ou amorce.

Les séquences sont généralement marquées pour obtenir des séquences utilisables pour de nombreuses application. Le marquage dés amorces ou des sondes selon invention est réaliste par des éléments radioactifs ou par des molécules non radioactives. Parmi les isotopes radioactifs utilisés, on peut citer le $^{32}P$, le $^{33}P$, le $^{35}S$, le $^{3}H$ ou le $^{125}I$. Les entités non radioactives sont sélectionnées parmi les ligands tels la biotine, l'avidine, la streptavidine, la dioxygénine, les haptène, les colorants, les agents luminescents tels que les agents radioluminescents, chémoluminescents, bioluminescents, fluorescents, phosphorescents.

L'invention comprend également une méthode de détection et/ou de dosage d'un polynucléotide selon l'invention, dans un échantillon biologique, **caractérisé en ce qu**'il comporte les étapes suivantes : (i) d'isolement de l'ADN à partir de l'échantillon biologique à analyser, ou obtention d'un ADNc à partir de l'ARN de l'échantillon biologique ; (ii) d'amplification spécifique de l'ADN codant pour le polypeptide selon l'invention à l'aide d'amorces ; (iii) d'analyse des produits d'amplification. C'est également un objet de l'invention de fournir une trousse pour la détection et/ou le dosage d'un acide nucléique selon l'invention, dans un échantillon biologique, **caractérisé en ce qu**'il comprend les éléments suivants : (i) un couple d'amorces nucléiques selon l'invention, (ii) les réactifs nécessaires pour effectuer une réaction d'amplification d'ADN, et éventuellement (iii) un composant permettant de vérifier la séquence du fragment amplifié, plus particulièrement une sonde selon l'invention.

L'invention comprend aussi une méthode de détection et/ou de dosage d'acide nucléique selon l'invention, dans un échantillon biologique, **caractérisé en ce qu**'il comporte les étapes suivantes : (i) de mise en contact d'un polynucléotide selon l'invention avec un échantillon biologique ; (ii) de détection et/ou de dosage de l'hybride formé entré ledit polynucléotide et l'acide nucléique de l'échantillon biologique. C'est également un objet de l'invention de fournir une trousse pour la détection et/ou le dosage d'acide nucléique selon l'invention, dans un échantillon biologique, **caractérisé en ce qu**'il comprend les éléments suivants : (i) une sonde selon l'invention, (ii) les réactifs nécessaires à la mise en *oeuvre* d'une réaction d'hybridation, et/ou le cas échéant, (iii) un couple d'amorces selon l'invention, ainsi que les réactifs nécessaires à une réaction d'amplification de l'ADN.

De préférence, l'échantillon biologique selon l'invention dans lequel sont réalisés la détection et le dosage est constitué par un milieu de culture, un broyat cellulaire, un fluide corporel, par exemple un sérum humain ou animal, du sang, du lait.

La présente invention concerne également les vecteurs recombinants de clonage et/ou d'expression comprenant un polynucléotide selon l'invention et/ou exprimant un polypeptide selon l'invention. Une telle cellule hôte est également un objet de l'invention.

De préférence, les vecteur recombinants selon l'invention sont :

- le vecteur appelé pLH2 comprenant le polynucléotide SEQ ID N°1 tel que présent dans la souche bactérienne déposée à là CNCM le 30 mai 2001 sous le N°I-2676 ; le plasmide pLH2 contient un fragment Alu **I** de 1,4 kb contenant le gène codant pour la N-désoxyribosyltransférase dé type II de *Lactobacillus helveticus* CNRZ32 cloné dans le site SmaI du plasmide pBAM3 ; le plasmide pLH2, qui exprime cette enzyme, est propagé dans la souche PAK6 auxotrophe pour la guanine ;
- le vecteur appelé pLH4 comprenant le polynucléotide SEQ ID N°3 tel que présent dans la souche bactérienne déposée à la CNCM le 30 mai 2001 sous lé N°I-2677 ; le plasmide pLH4 contient un fragment AluI de 1,6 kb contenant le gène codant pour la N-désoxyribosyltransférase de type I de *Lactobacillus helveticus* CNRZ32, cloné dans le site SmaI du plasmide pBAM3. Le plasmide pLH4, qui exprime cette enzyme, est propagé dans la souche PAK6 auxotrophe pour la guanine ;
- le vecteur rappelé pLF6 comprenant le polynucléotide SEQ ID N°5 tel que présent dans la souche bactérienne déposée à la CNCM le 30 mai 2001 sous le N°I-2678 ; le plasmide pLF6 contient un fragment AluI de 1,36 kb contenant le gène codant pour la N-désoxyribosyltransférase de type II de *Lactobacillus fermentum* CIP102780T. Le plasmide pLF6, qui exprime cette enzyme est propagé dans la souche PAK6 auxotrophe pour la guanine ;
- le vecteur appelé pLA comprenant le polynucléotide SEQ ID N°11 tel que présent dans la souche bactérienne déposée à la CNCM le 21 juin 2001 sous le N° I-2689 ; le plasmide pLA correspond au plasmide pSU19 est cloné aux sites PstI et BamHI un insert contenant le gène codant pour la N-désoxyribosyltransférase de type II *de Lactobacillus acidophilus* CNRZ 1296. Le plasmide est propagé dans la souche d'*Escherichia coli* TG-I.

Les vecteurs selon l'invention comportent les éléments nécessaires à l'expression, et notamment, de préférence un promoteur, des signaux d'initiation et de terminaison de la traduction, ainsi que des régions appropriées de régulation de la transcription. Ils doivent pouvoir être maintenus due façon stable dans la cellule et peuvent éventuellement posséder des signaux particuliers spécifiant la sécrétion de la protéine traduite.

Les différents signaux de contrôle sont choisis en fonction de l'hôte cellulaire utilisé. A cet effet, les séquences d'acide nucléique selon l'invention peuvent être insérées dans des vecteurs à réplication autonome au sein de l'hôte choisi, ou des vecteurs intégratifs de l'hôte choisi. Parmi les systèmes à réplication autonome, on utilise de préférence en fonction de la cellule hôte, des systèmes de type « plasmide », « cosmide », « phagemide » ou « mini-chromosome », ou des systèmes de type viral, les vecteurs viraux pouvant notamment être des adénovirus (Perricaudet *et al.*, 1992), des rétrovirus, des lentivirus, des poxvirus ou des virus herpétiques (Epstein *et al.*, 1992). L'homme du métier connaît les technologies utilisables pour chacun de ces systèmes. Lorsque l'on souhaite intégration de la séquence dans les chromosomes de la cellule hôte, on peut utiliser par exemple des systèmes de type plasmidique ou viral ; de tels virus sont, par exemple, les rétrovirus (Temin, 1986), ou les AAV (Carter, 1993).

Parmi les vecteurs non viraux, on préfère les polynucléotide nus tels que l'ADN nu ou l'ARN nu selon la technique développée par la société VICAL, les chromosomes artificiels de bactérie (BAC, "bacterial artificial chromosome"), les chromosomes artificiels de levure (YAC, "yeast artificial chromosome") pour l'expression dans la levure, les chromosomes artificiels de souris (MAC, "mousse artificial chromosome") pour l'expression dans les cellules murines et de manière préférée les chromosomes artificiels d'homme (HAC, "human artificial chromosome") pour l'expression dans les cellules humaines.

De tels vecteurs sont préparés selon les méthodes couramment utilisées par l'homme du métier, et les clones en résultant peuvent être introduits dans un hôte approprié par des méthode standard, telles que par exemple la lipofection, l'électroporation, le choc thermique, la transformation après perméabilisation chimique de la membrane, la fusion cellulaire.

L'invention comprend en outre les cellules hôtes, notamment les cellules eucaryotes et procaryotes, transformées par les vecteurs selon l'invention. Parmi les cellules utilisables aux sens de la présente invention, on peut citer les bactéries et les levures. Selon un mode de réalisation préférée de l'invention, la bactérie est choisie parmi le groupe composé de *Lactobacillus fermentum, Lactobacillus acidophilus, lactobacillus amylovorus, Lactobacillus crispatus, Lactobacillus helveticum, LactoBacillus lactis, Escherichia coli, Bacillus subtilis, Campylobacter pylori, Helicobacter pylori, Agrobacterium tuméfaciens, Staphylococcus aureus, Thermophilus aquaticus, Azorhizobium caulinodans, Rhizobium leguminosarum,*

*Neisseria gonorrhoeae, Neisseria meningitis*. Selon un mode préféré de réalisation, de l'invention, la bactérie est *Lactobacillus*. Selon un mode préféré, il s'agit de :

- la bactérie transformée par le plasmide pLH2 comprenant le polynucléotide SEQ ID N°1 telle que déposée à la CNCM le 30 mai 2001 sous le N°1-2676 ;
- la bactérie transformée par le plasmide pLH4 comprenant le polynucléotide SEQ ID N°3 telle que déposée à la CNCM le 30 mai 2001 sous le N°1-2677 ;
- la bactérie transformée par le plasmide pLF6 comprenant le polynucléotide SEQ ID N°5 telle que déposée à la CNCM le 30 mai 2001 sous le N°I-2678 ;
- la bactérie transformée par le plasmide pLA comprenant le polynucléotide SEQ ID N°11 telle que déposée à la CNCM le 21 juin sous le N°I-2689.

Selon un autre mode préféré de réalisation la bactérie est *Escherichia coli*. Selon un autre mode de réalisation de l'invention, la cellule est une levure qui est de préférence *Saccharomyces cerevisiae, Saccharomyces pombe, Candida albicans*.

Parmi les cellules hôte selon l'invention, il convient également de citer les cellules d'insectes, les cellules animales ou végétales.

De préférence, la cellule selon l'invention est dépourvue d'activité enzymatique susceptible de dégrader ledit désoxyribonucléoside précurseur ou ledit désoxyribonucléoside obtenu par réaction bioenzymatique catalysée par un polypeptide selon l'invention. Alternativement, ladite cellule hôte peut être pourvue d'activités bio-enzymatiques additionnelles destinées à transformer le désoxyribonucléoside précurseur, et/ou le désoxyribonucléoside obtenu par la réaction bioenzymatique catalysée par le polypeptide selon l'invention. Parmi ces activités bio-enzymatiques additionnelles, il convient de citer la phosphorylation, la sulfatation, l'acétylation, la succinylation, la méthylation.

La séquence d'acide nucléique codant pour les N-désoxyribosyltransférases selon l'invention est soit naturellement présente dans ladite cellule soit est introduite dans ladite cellule par les techniques de l'ADN recombinant connues de l'homme du métier. Selon un mode préféré de réalisation, la séquence d'acide nucléique introduite dans ladite cellule par les techniques de l'ADN recombinant et qui code pour une N-désoxyribosyltransférase selon l'invention est hétérologue. On entend désigner par séquence d'acide nucléique hétérologue, une séquence d'acide nucléique qui n'est pas présente naturellement dans la cellule selon l'invention.

La présente invention concerne également les organismes métazoaires, végétaux ou animaux, de préférence les mammifères, excepté l'Homme, comprenant une desdites cellules transformées selon l'invention. Parmi les animaux selon l'invention, on préfère les rongeurs, en particulier les souris, les rats ou les lapins, exprimant au moins un polypeptide selon l'invention.

Les cellules de préférence bactériennes, ou fongiques notamment de levure, ainsi que les organismes métazoaires selon l'invention sont utilisables dans une méthode de production de N-désoxyribosyltransférase selon l'invention. La méthode de production d' un polypeptide de l'invention sous forme recombinant, elle-même comprise dans la présente invention, se **caractérise en ce que** l'on cultive les cellules transformées, notamment les cellules de la présente invention, dans des conditions permettant l'expression et éventuellement la sécrétion d'un polypeptide recombinant codé par une séquence d'acide nucléique selon l'invention, et que l'on récupère ledit polypeptide recombinant. Les polypeptides recombinants susceptibles d'être obtenus par cette méthode de production font également partie de invention. Ils peuvent se présenter sous forme glycosylée ou non glycosylée et peuvent présenter ou non la structure tertiaire de la protéine naturelle. Les séquences des polypeptides recombinant peuvent être également modifiées afin d'améliorer leur solubilité, en particulier dans les solvants aqueux. De telles modification sont connues de l'homme du métier comme par exemple la délétion de domaines hydrophobes ou la substitution d'acides aminés hydrophobes par des acides aminés hydrophiles.

Ces polypeptides peuvent être produits à partir des séquences d'acide nucléique définies ci-dessus, selon les techniques de production de polypeptides recombinants connues de l'homme du métier. Dans ce cas, la séquence d'acide nucléique utilisée est placée sous le contrôle de signaux permettant son expression dans un hôte cellulaire.

Un système efficace dé production d'un polypeptide recombinant nécessite de disposer d'un vecteur et d'une cellule hôte selon l'invention. Ces cellules peuvent être obtenues par l'introduction dans des cellules hôtes d'une séquence nucléotidique insérée dans un vecteur tel que défini ci-dessus, puis la mise en culture desdites cellules dans des conditions permettant la réplication et/ou l'expression de la séquence nucléotidique transfectée.

Les procédés utilisés pour la purification d'un polypeptide recombinant sont connus de l'homme du métier. Le polypeptide recombinant peut être purifié à partir de lysats et extraits cellulaires, du surnageant du milieu de culture, par des méthodes utilisées individuellement ou en combinaison, tels que le fractionnement, les méthodes de chromatographie, les techniques d'immunoaffinité à l'aide d'anticorps monoclonaux ou polyclonaux spécifiques, etc. Une variante préférée consiste à produire un polypeptide recombinant fusionné à une protéine « porteuse » (protéine chimère). L'avantage de ce système est qu'il permet une stabilisation et une diminution de la protéolyse du produit recombinant, une augmentation de la solubilité au cours de la renaturation *in vitro* et/ou une simplification de la purification lorsque le partenaire de fusion possède une affinité pour un ligand spécifique.

Les polypeptides selon la présente invention peuvent aussi être obtenus par synthèse chimique en utilisant l'une des nombreuses synthèses peptidiques connus, par exemple les techniques mettant en oeuvre des phasmes solides ou des techniques utilisant des phases solides partielles, par condensation de fragments ou par une synthèse en solution classique. Les polypeptides obtenus par synthèse chimique et pouvant comporte des acides aminés non naturels correspondants sont également compris dans l'invention.

Les polypeptides selon l'invention permettent de préparer des anticorps monoclonaux ou polyclonaux. C'est donc également un des objets de la présente invention de fournir un anticorps monoclonal ou polyclonal et ses fragments, **caractérisés en ce qu**'ils lient sélectivement et/ou spécifiquement un polypeptide selon l'invention. Les anticorps chimériques, les anticorps humanisés et les anticorps simple chaîne font également partie de l'invention. Les fragments d'anticorps selon l'invention sont de préférence des fragments Fab, F(ab')2, Fc ou Fv. Les anticorps polyclonaux pourront être préparés, par exemple par immunisation d'un animal, en particulier une souris, avec un polypeptide selon l'invention associé à un adjuvant de la réponse immunitaire, puis purification des anticorps spécifique contenus dans le sérum des animaux immunisés sur une colonne d'affinité sur laquelle a préalablement été fixé le polypeptide ayant servi d'antigène. Les anticorps polyclonaux selon l'invention peuvent aussi être préparés par purification sur une colonne d'affinité, sur laquelle a préalablement été immobilisé un polypeptide selon l'invention. Les anticorps monoclonaux pourront avantageusement être préparés à partir d'hybridomes selon la technique décrite par Kohler et Milstein en 1975.

Selon un mode particulier de réalisation de l'invention, l'anticorps est capable d'inhiber l'interaction entre la N-désoxyribosyltransférase de l'invention et son substrat afin d'altérer la fonction physiologique dudit polypeptide N-désoxyribosyl transférase.

L'invention concerne également des méthodes pour la détection et/ou la purification d'un polypeptide selon l'invention, **caractérisés en ce qu**'elles mettent en oeuvre un anticorps selon l'invention. L'invention comprend en outre des polypeptides purifiés, **caractérisés en ce qu**'ils sont obtenus par une méthode selon l'invention.

Par ailleurs, outre leur utilisation pour la purification des polypeptides, les anticorps de l'invention, en particulier les anticorps monoclonaux, peuvent également être utilisés pour la détection de ces polypeptides dans un échantillon biologique.

Pour ces différentes utilisations, les anticorps de l'invention pourront également être marqués de la même manière que décrit précédemment pour les sondes nucléiques de l'invention et de manière préférée avec un marquage de type enzymatique, fluorescent ou radioactif.

Les anticorps de l'invention constituent également un moyen d'analyse de l'expression de polypeptide selon l'invention, par exemple par immunofluorescence, marquage à l'or, immunoconjugués enzymatiques. Plus généralement, les anticorps de l'invention peuvent être avantageusement mis en oeuvre dans toute situation où l'expression d'un polypeptide selon l'invention, normal ou muté, doit être observée, et plus particulièrement en immunocytochimie, en immunohistochimie ou dans des expérience de « western blotting ». Ainsi, un procédé de détection d'un polypeptide selon l'invention dans un échantillon biologique, comprenant les étapes de mise en contact de l'échantillon biologique avec un anticorps selon l'invention et de mise en évidence du complexe antigène-anticorps formé est également un objet de l'invention.

C'est également un des objets de la présente invention de fournir un procédé de synthèse enzymatique *in vitro* ou *in vivo* de désoxyribonucléotides **caractérisé en ce qu**'il comprend au moins une étape réactionnelle catalysée par au moins une N-désoxyribosyltransférase selon l'invention. Le procédé selon invention se **caractérisée en ce que** la dite N-désoxyribosyl transférase catalyse l'échange d'une première nucléobase présente dans un désoxyribonucléoside par une seconde nucléobase.

Selon un mode préféré de réalisation de l'invention, la dite seconde nucléobase est sélectionnée dans le groupe composé des purines liées par N9, des pyrimidines liées par N1, des azines liées par N1, des imidazoles liées par N1, lesdites secondes nucléobases pouvant porter des substitutions des hydrogènes aux positions non liées. De préference, ladite seconde nucléobase est sélectionnée dans le groupe composé de la 6-méthyl purine, 2-amino-6-méthylmercaptopurine, 6-diméthylaminopurine, 5-azacytidine, 2,6-dichloropurine, 6-chloroguanine, 6-chloropurine, 6-aza-thymine, 5-fluoro-uracile, éthyl-4-amino-5-imidazole carboxylate, imidazole-4-carboxamide et 1,2,4-triazole-3-carboxamide. La dite première nucléobase est quant à elle, sélectionnée de préference dans le groupe composé de l'adénine, la guanine, la thymine, l'uracile et l'hypoxanthine. Ces listes ne sont pas exhaustives, et il est évident que des analogues naturels ou non-naturels de nucléobases peuvent être employés dans la présente invention comme substrat d'une N-désoxyribosyl transférase de l'invention.

Facultativement, le procédé *in vivo* selon l'invention se **caractérise en ce qu**'il comprend en outre l'étape d'introduire dans la cellule hôte la première nucléobase présente dans un désoxyribonucléoside.

Facultativement, le procédé *in vivo* selon l'invention se **caractérise en ce qu**'il comprend en outre l'étape d'introduire dans la cellule hôte la seconde nucléobase présente dans une désoxyribonucléoside.

Facultativement, le procédé *in vivo* selon l'invention se **caractérise en ce qu**'il comprend en outre l'étape d'introduire dans la cellule hôte la première nucléobase présente dans un désoxyribonucléoside et la seconde nucléobase de manière simultanée et/ou décalée dans le temps.

Les désoxyribonucléosides susceptibles d'être produit en grande quantité et de manière peu onéreuse par la méthode de biosynthèse selon l'invention constituent donc des composés d'intérêt destinés au traitement préventif ou curatif de pathologies humaines ou animales, tumorales, virales telles que le SIDA (syndrome d'immunodéficience humaine acquise), bactériennes, parasitaires ou fongiques, Alternativement, ces désoxyribonucléosides susceptibles d'être produit en grande quantité et de manière peu onéreuse par la méthode de biosynthèse selon l'invention constituent des herbicides et des insecticides,

La présente invention fournit également un procédé de criblage nutritionnel destiné à isoler des désoxyribosyltransférases, de préference les polypeptides selon l'invention mais également leurs homologues ou leurs mutants. Ce premier crible selon l'invention comprend les étapes :

(i) (facultativement) obtention d'une souche bactérienne, telle *Escherichia coli*, auxotrophe en guanine. De préférence cette souche est incapable de pousser en présence de désoxyguanosine comme source de guanine. De manière préférée, il s'agit de la souche PAK 6.
(ii)transfert d'ADN exogène, de préférence sous la forme d'un vecteur d'expression, dans la bactérie, l'ADN exogène étant susceptible de comprendre une séquence codant pour une désoxyribosyltransférase.
(iii)culture des bactéries obtenues à l'étape (ii) sur un milieu contenant de la désoxyguanosine.
(iv)isolement de l'ADN exogène transféré dans les bactéries de l'étape (iii) qui se sont développées sur le milieu contenant de la désoxyguanosine.

La présente invention fournit également un crible nutritionnel pour distinguer les activités désoxyribosyltransférases I et II, de préference notamment pour distinguer entre les polypeptides ntd et ptd selon l'invention. Ce second crible comprend les étapes de :

(i) obtention d'une souche bactérienne telle par exemple *Escherichia coli*, auxtrophe pour la guanine et la thymidine. De préférence cotte souche est incapable de pousser en présence de guanine et de thymidine. De manière préférée, il s'agit de la souche PAK 26 (Δ guaBguaA :: Δdeo-11 ΔthyA :: erm Δ(udp-metE)zif9 :: Tn10) est auxtrophe pour la méthionine, la guanine et la thymidine).
(ii) transfert de l'ADN exogène, de préférence sous la forme d'un vecteur d'expression dans la bactérie, l'ADN exogène étant susceptible de comprendre une séquence codant pour une désoxyribosyltransférase I ou II.
(iii)culture des bactéries obtenues à l'étape (ii) sur un milieu contenant de la désoxyguanosine puis détermination si les bactéries poussent ou non. Si les bactéries poussent, alors l'ADN exogène code pour une activité désoxyribosyltransférase II qui s'exprime dans la dite bactérie. Si les bactériens ne poussent pas, alors l'ADN exogène est susceptible de coder pour une activité désoxyribosyltransférase I.

D'autres caractéristiques et avantages de l'invention apparaissent dans la suite de la description avec les exemples représentés ci-aprés.

**EXEMPLES**

**1. MATERIEL ET METHODES**

**1.1. Souches et conditions de culture :**

Les souches de bactéries lactiques utilisées proviennent de la collection CNRZ (Centre National de Recherche Zootechnique), Unité de Recherches Laitières et Génétique Appliquée, INRA, Jouy en Josas. Elles sont cultivées en bouillon MRS (de Man et coll., J. Appl. Bacteriol., 23 :130-135, 1960) et incubées à 30°C, 37°C ou 42°C selon les espèces. La souche d'Escherichia coli TG1, fournie par Stratagène, est cultivée dans du LB (Luria broth base 10g/L, Agar-agar 16g/L) sous agitation et à 37°C.

**1.2. Préparation de l'ADN cellulaire total des bactéries lactiques :**

Les cultures en fin de phase exponentielle sont centrifugées pendant 5 minutes à 13000 g. Le culot correspondant à une culture de 2 ml est repris dans 200 μl de TES (Tris 50 mM, pH8, EDTA 10 mM, pH8, saccharose 250 mM) contenant 20 μg/ml de lysozyme et 50 U/ml de mutanolysine (Sigma). Après une incubation d'une heure à 37°C, la clarification de la préparation est obtenue en ajoutant 60 μl de SDS 20%.

L'extraction des acides nucléiques est effectuée en ajoutant au lysat 500 μl de phénol saturé en eau, pH8, additionné d'hydroxyquinoline 0.1% et 100 μl d'un mélange de chloroforme-alcool isoamylique (24/1, V/V). La solution est homogénéisée puis centrifugée 10 minutes à 13000 g et à température ambiante. La phase supérieure, limpide et contenant les acides nucléiques est gardée. Sur cette dernière, l'extraction est répétée trois fois afin d'éliminer lés constituants cellulaires indésirables. Les traces de phénol sont éliminées en ajoutant 500 μl de chloroforme-alcool isoamylique à la phase aqueuse. Après homogénéisation et centrifugation pendant 3 minutes à 15000 g et à 4°C, les acides nucléiques contenus dans la phase aqueuse supérieure sont précipités par addition d'un volume d'isopropanol froid. Après une incubation d'une heure à -20°C, une centrifugation est effectuée pendant 20 minutes à 15000 g et à 4°C. L'isopropanol est. éliminé et remplacé par 500 μl d'éthanol 70%. Une dernière centrifugation de 10 minutes à 15000 g et à 4°C permet de récupérer un culot d'acides nucléiques. Celui-ci est mis à sécher dans un évaporateur et remis en suspension dans 200 μl d'eau stérile contenant 10 μl d' ARNase à 10 μg/μl. Après 15 minutes d'incubation à 37°C pour faire agir l'enzyme dégradant les ARN, 10 μl de la solution d'ADN sont mis à migrer par électrophorèse dans un gel d'agarose 0.8% afin d'en évaluer la concentration et la qualité.

**1.3. Réaction de polymérisation en chaîne de l'ADN (PCR) :**

Les réactions de polymérisation en chaîne (PCR) sont effectuées dans un volume réactionnel de 100 μl contenant 20 à 100 ng d'ADN, 0,5 μM d'amorces, 200 μM des dNTPs (dATP, dGTP, dGTP, dTTP), dans un tampon Tris-HCl pH 9 à 10 mM, KC1 à 50 mM, $MgCl_2$ à 1,5 mM, BSA à 0,002% ainsi que 2,5 unités de la polymérase Taq. Trente cycles d'amplification ont été appliqués (Gene Amp PCR systems 2400, Perkin Elmer). Les inventeurs ont défini deux amorces ntd 1 (SEQ ID N° 15) et ntd2 (SEQ ID N° 16) à partir de la séquence ntd de *Lactobacillus leichmanii* décrite par Huck (communication personnelle) :

| | | | |
|---|---|---|---|
| ntd 1 | 5'-AGA CGA TCT ACT TCG GTG-3' | 18 bases | Tm= 54°C |
| ntd 2 | 5'-ACG GCA CCT TCG TAG AAG-3' | 18 bases | Tm= 56°C |

**1.4. Hybridation de type Southern :**

***Restriction enzymatique des ADN.*** Les ADN totaux sont digérés par une ou plusieurs enzymes de restriction. Les enzymes utilisées sont : BamHI, BglII, ClaI, EcoRI, HindIII, HpaI, NcoI, NotI, PstI, XbaI, XhoI (Bio-Lab). La digestion est réalisée dans un volume final de 40 μl contenant 70 U d'enzyme, 4 μl de tampon NEB (Bio-Lab) 10X et 4 à 8 μg

d'ADN. L'incubation s'effectue pendant lh30 à 37°C.

***Transfert de l'ADN sur une membrane.*** Les fragments d'ADN totaux issus de la digestion enzymatique sont séparés à l'aide d'un gel d'agarose à 0.7%. Après migration, le gel d'agarose est placé sous agitation dans une solution de dépurination (HCl 0.25N) pendant 30 minutes. Ce procédé permet ainsi le transfert des fragments d'ADN supérieur à 10 kbs. Après rinçage à l'eau, l'ADN est dénaturé en plaçant le gel pendant 40 minutes dans une solution de NaCl 5M, NaOH 0,5M. Le gel est rincé à l'eau puis incubé de nouveau 30 minutes dans une solution de neutralisation, NaCl 1,5M, Tris HCl 0,5M; pH 7,5. Les ADN sont transférés par capillarité sur une membrane de nylon chargée positivement (Hybond N+, Amersham). Ils sont élués par un flux ascendant de SSC 20X (citrate trisodique 0,3M ; NaCL 3M ; pH7). Après le transfert, les ADN sont fixés de manière covalente sur la membrane à l'aide d'un appareil UV Stratalinker 2400 (Stratagene).

***Préparation de la sonde.*** La sonde utilisée est un fragment interne du gène ntd de *Lactobacillus helveticus* amplifié par PCR. La sonde est purifiée à l'aide du kit Wizard (Promega) afin d'éliminer les amorces PCR. La concentration de sonde nécessaire est de 10 ng/μl. Le marquage de l'ADN est réalisé en utilisant le kit de marquage ECL (Amersham). Pour ce faire, l'ADN est dénaturé par chauffage 5 minutes à 100°C et immédiatement refroidi dans la glace. Un volume de réactif de marquage (peroxydase) puis un volume de solution de glutaraldhéhyde sont additionnés. Cette solution est incubée pendant 10 minutes à 37°C pour fixer de manière covalente la peroxydase à l'ADN.

***Hybridation et révélation.*** Après une préhybridation d'une heure à 42°C dans du tampon d'hybridation, la membrane est hybridée pendant 16h à 42°C en présence de la sonde marquée. Afin d'éliminer la sonde fixée de manière non spécifique, la membrane est lavée pendant 20 minutes à 42°C dans deux bains successifs de tampon: urée 6M - SDS 0,4% - SSC 0,5X, puis rincée pendant 5 minutes dans deux bains successifs de tampon: citrate de sodium 0, 3M - NaCl 3M pH 7. La révélation est effectuée par autoradiographie selon le protocole du kit ECL. Un premier réactif de révélation contenant du peroxyde d'hydrogène est réduit par la peroxydase fixée sur la sonde. Puis le luminol contenu dans un deuxième réactif de révélation est alors oxydé, produisant de la lumière qui impressionne le film autoradiographique.

**1.5. Clonage de fragments PCR :**

Les gènes homologues à ntd amplifiés par PCR sont insérés dans le plasmide vecteur pBluescript II SK+ d'*E. coli* TG1 (Stratagene). Ce plasmide est auparavant restreint en son site unique par l'enzyme EcoRV (Gibco-BRL) qui crée des extrémités franches. Le mélange de digestion est effectué dans un volume de 30 μl, contenant 4 μl d'ADN. Les fragments des ADN amplifiés à cloner doivent avoir leurs extrémités 5' et 3' franches afin de permettre le clonage. La préparation des extrémités franches de 50 μl de produits PCR purifiés à l'aide du kit Wizard (Promega) est réalisée dans un volume réactionnel de 100 μl contenant 3,6 unités d'ADN polymérase du phage T4 (Bio-Lab) et 6 unités d'ADN polymérase I (ou fragment de Klenow) (Bio-Lab), n'ayant pas d'activité exonucléasique 5' > 3'. La polymérisation se fait pendant 20 minutes à 11°C puis les enzymes sont inactivées après 10 minutes à 75°C. L'ADN est ensuite précipité en présence de deux volumes d'éthanol 100%, de glycogène et de 10 % d'acétate de sodium 3M, pH 4,8. Le mélange est placé pendant une heure à -20°C puis centrifugé 20 minutes à 15000 g. Le culot est rincé avec 250 μl d'éthanol à 70 %, centrifugé à nouveau 10 minutes à 15000 g, séché dans un évaporateur et remis en suspension dans 20 μl d'eau stérile.

L'ADN restreint du plasmide pBS-SK+ et le fragment amplifié sont mis à co-migrer sur un gel d'agarose à 0,7 % afin d'évaluer leurs concentrations respectives : le nombre de molécules du fragment à cloner doit être trois à quatre fois supérieur à celui du plasmide. La ligation se fait dans un volume de 10 μl contenant 60 ng d'insert, 26 ng de plasmide pBS-SK+ restreint, 2 unités de ligase (T4 DNA ligase, Boehringer-Mannheim), pendant la nuit à 16°C. Les produits de ligation sont dialysés sur un filtre Milipore 0,025 μm de manière à enlever les sels et à éviter ainsi les arcs électriques lors de l'électroporation.

**1.6. Transformation :**

***Préparation des cellules éleotxo-aonvétentes de la souche TG1 d'E. coli.*** A partir de 5 ml d'une culture de nuit à 37°C sous agitation, 500 ml de milieu LB sont inoculés. La culture est placée sous agitation à 37°C jusqu'à atteindre une $D.O._{600nm}$ = 1. Elle est ensuite refroidie pendant 2 heures dans la glace puis centrifugée pendant 10 minutes à froid à 5000 rpm. Le surnageant est éliminé, le culot est repris dans 400 ml d'eau froide. Cette préparation est centrifugée pendant 10 minutes à froid et à 5000 trs/mn. Le culot obtenu est de nouveau repris dans 250 ml d'eau froide. A la suite d'une centrifugation de 10 minutes, le culot est repris dans 25 ml d'eau froide puis les cellules sont remises en suspension dans un volume final de 1ml de glycérol 10%, et réparties en aliquot avant d'être congelées rapidement dans l'azote liquide.

***Transformation par électroporation et sélection des clones***. Les cellules électro-compétentes conservées à 80°C sont décongelées dans la glace puis mises en contact avec 5 μl de mélange de ligature dans une cuvette d'électroporation. Le Gene-Pulser (Bio-Rad) est réglé à 200 Volts, 25 mF, 250 Ohms. Les cellules sont ensuite soumises à l'électroporation. On ajoute 1ml d'une solution de SOC (bactopeptone 20g/L, yeast extract 5g/L, NaCl 5M 2ml/L, KCl 1M 2,5 ml/L, $MgCl_2$, 1M 10ml/L, $MgSO_4$ 1M 10ml/L), contenant 0.4% de glucose à la suspension cellulaire qui est mise à incuber à 37°C pendant une heure. Les cellules sont ensuite étalées sur un milieu sélectif LB -Xgal (5-bromo-4-chloro-3-indolyl-β-D-galatoside à 1μg/mL) -IPTG (isopropythio-β-D-galactoside, 1μg/ml) -Ampicilline (50 μg/ml), et incubées à 37°C pendant la nuit.

***Extraction rapide d'ADN plasmidique des clones de E. coli recombinants par lyse alcaline.*** Les cellules de E. coli transformées et cultivées en milieu LB contenant de l'ampicilline (100 μg/ml) sont récoltées par centrifugation à 15000 g pendant 10 minutes à 4°C. Elles sont resuspendues dans 100 μl d'une solution Saccharose 50 mM, Tris-HCl 25mM, pH 8, EDTA 10 mM, pH 8. La lyse alcaline et la dénaturation de l'ADN se fait par ajout de 200 μl de NaOH 0.2N, SDS 1%. L'ensemble est laissé 1 minute à température ambiante après avoir ajouté 200 μl de chloroforme. Puis, 150 μl d'une solution d'acétate de potassium 5M, acide ascétique glacial sont additionnés. L'ensemble est centrifugé pendant 15 min à 13000 g à 4°C. La phase aqueuse contenant l'ADN est précipitée en présence de 2 volumes d'éthanol 100% puis centrifugée pendant 20 minutes à 13000 g à 4°C. Le culot est lavé à l'éthanol 70%, centrifugé 10 minutes à 13000 g puis remis en suspension dans 30 μl d'eau stérile contenant de l'ARNase à 10 ng/ml.

**1.7. PCR inverse :**

La PCR inverse permet d'amplifier les régions flanquant un fragment d'ADN de séquence connue. Cette technique se déroule en trois étapes :

***Digestion de la matrice d'ADN.*** La matrice d'ADN est digérée par une ou deux enzymes de restriction choisies de telle sorte qu'elles ne coupent pas dans la séquence connue du gène et qu'elles permettent d'obtenir un fragment de taille adaptée (1 à 3 kb). Afin de choisir une enzyme adaptée, l'ADN total est digéré séparément par plusieurs enzymes. Puis des hybridations de type Southern sont réalisées en utilisant comme sonde le fragment d'ADN de séquence connue. Les digestions pour lesquelles la sonde hybride avec un fragment de taille de 1 à 3 kb, sont utilisées pour la PCR inverse. Les fragments d'ADN obtenus par digestion sont circularisés. Pour cela, 100 unités de T4 ADN ligase et 100 μl de tampon de ligation sont ajoutés à 4 à 8 μg d'ADN dans un volume final de 1ml. Le mélange de ligation est mis à incuber à 15°C pendant la nuit. L'ADN ligaturé est ensuite précipité avec 100 μl d'acétate de sodium 3M, pH 4.8, 700 μl d'isopropanol et 2 μl de glycogène puis centrifugé pendant 30 minutes à 13000 g, à 4°C. Le culot est rincé dans 300 μl d'éthanol 70% et centrifugé 10 minutes à 13000 g, à 4°C. Le culot est repris dans 25 μl d'eau ultrapure.

***Amplification des ADN circularisés en utilisant des amorces divergentes.*** Les réactions de polymérisation en chaîne sont effectuées dans un volume réactionnel de 100 μl contenant 20 à 100 ng d'ADN, 0,5 μM d'amorces, 200 μM des dNTPs (dATP, dCTP, dGTP, dTTP), dans un tampon Tris-HCl pH 9 à 10 mM, KC1 à 50 mM, MgC12 à 1,5 mM BSA à 0,002% ainsi que 2,5 unités de la polymérase Taq.

L'amplification est effectuée dans les conditions suivantes :

| | | |
|---|---|---|
| 94°C | 3 minutes | |
| 94°C | 30 secondes | 25 cycles |
| 50 à 60°C (selon le Tm des amorces utilisées) | 1 minute | |
| 72°C | 3 minutes | |

(Gene Amp PCR systems 2400, Perkin Elmer).

***Séquençage.*** Les fragments PCR sont purifiés par le kit Wizard (Promega) afin d'éliminer les oligonucléotides non incorporés, les sels et la Taq polymérase. Le séquençage est réalisé à l'aide d'un séquenceur automatique 373A (Applied Biosystems-Perkin Elmer) en utilisant un kit ABI PRISM Dye Terminator (Perkin Elmer), basé sur l'incorporation de désoxynucléotides phosphate fluorescents lors de la phase d'élongation des amorces. Les réactions de séquences sont effectuées dans un volume réactionnel de 20 μl contenant 30 ng d'ADN, 4 μl de DyeT Mix (Perkin Elmer Biosystems) et 0.1mM d'amorce.

Cycle :

| | | | |
|---|---|---|---|
| 96°C | 1 minute | | |
| 96°C | 10 secondes | } | 25 cycles |
| 50°C | 5 secondes | } | |
| 60°C | 4 minutes | } | |

A chaque réaction de séquence sont ajoutés 20 µl d'acétate de sodium 3M, pH 4.6, 50 µl d'éthanol à 95% et 1 µl de glycogène. La solution est laissée 15 minutes à température ambiante puis centrifugée 20 minutes à 13000 g. Le culot est ensuite rincé avec 250 µl d'éthanol 70% puis centrifugé pendant 10 minutes à 13000 g. Le culot est ensuite repris dans 6 µl de bleu de séquence (formamide 83%, EDTA 8.3mM, bleu dextran 2000000 (Sigma) 0.5%). Les échantillons sont dénaturés pendant 3 minutes à 90°C et 3 µl sont déposés sur gel d'acrylamide 4%.

## 2. UN CRIBLE NUTRITIONNEL UNIQUE POUR LES DEUX CLASSES DE N-DBSOXYRIBOSYLTRANSFERASE CHEZ *ESCHERICHIA COLI*.

Un crible fonctionnel permettant de sélectionner la production de guanine a été établi chez *E. coli*, en délétant les deux gènes de l'opéron *guaBA* qui commande la conversion de IMP en XMP puis en GMP ainsi que ceux de l'opéron *deoCABD* qui commande la dégradation des désoxynucléosides, pour donner la souche PAK 6. Le génome d'*E. coli* spécifie une activité permettant de convertir la base G en GMP (guanine phosphoribosyltranférase codée par le gène *gpt*), ainsi qu'une activité permettant de libérer la base G du désoxynucléoside dR-G (purine nucléoside phosphorylase codée par le gène *deoD,* dans l'opéron *deo*), La souche PAK 6 présente donc une exigence en guanine (G) qui ne pourra être satisfaite par l'apport de désoxyguanosine (dR-G). L'utilisation de désoxyguanosine (dR-G) pourra cependant être sélectionnée si une activité N-désoxyribosyltransférase s'exprime dans la souche PAK6, pour réaliser l'échange: dR-G + A <-> dR-A + G

Cet échange entre deux bases purines peut être catalysé par les deux classes d'enzyme. De fait, l'introduction du gène ntd de *L. leichmannii* dans la souche PAK 6 permet de satisfaire 1' exigence en guanine à l'aide de désoxyguanosine (dR-G) et d'adénine (A).

## 3. CLONAGE FONCTIONNEL DU GENE PTD DE L. HELVETICUS.

Des fragments d'ADN de taille comprise entre 1 et 2kb obtenus par digestion partielle (AluI) *de L. helveticus* CNRZ 32 furent ligaturés dans un plasmide ColE1 (de type pUC digéré par SmaI et déphosphorylé) et le mélange fut utilisé pour transformer la souche PAK6. Les clones transformants furent sélectionnés en milieu minéral glucose additionné de désoxyguanosine (dR-G) et d'adénine (A) à la concentration finale de 0,3mM.

L'un des clones transformants se révéla propager un plasmide contenant un insert commandant une activité N-désoxyribosyltransférase de classe I et déviant du profil de restriction du gène ntd de *L. helveticus*. La séquence de cet insert révéla un gène spécifiant un polypeptide de 167 acides aminés avec un poids moléculaire de 18790.70 Daltons présentant une similarité de 28,6% avec le polypeptide NTD de *L. leichmanii*. La séquence de ce gène désigné ptd dévie de celle des gènes ntd rendant leur hybridation impossible (7,5% d'identité).

Incidemment, le gène ntd de *L. helveticus* commandant une activité N-désoxyribosyltransférase de classe II put être une nouvelle fois isolé parmi les clones transformants sélectionnés.

## 4. CLONAGE FONCTIONNEL DU GENE NTD *L. FERMENTUM.*

Les mêmes opérations de clonage et de sélection nutritionnelle furent effectuées à partir de l'ADN génomique de la souche *L. fermentum* CIP 102980T. Les clones transformants sélectionnés se révélèrent propager un plasmide dont les inserts présentant des profils de restriction semblables commandaient une activité N-désoxyribosyltransférase de classe II. La séquence d'un de ces inserts révéla un gène spécifiant un polypeptide de 168 acides aminés avec un poids moléculaire de 18878.20 Daltons présentant une similarité de 32,9% avec le polypeptide NTD de *L. leichmanii* et de 36,7% avec le polypeptide PTD de *L. helveticus*. La séquence de ce gène dévie de celle des gènes ntd et ptd rendant leur hybridation impossible. Le polypeptide NTD de *L. fermentum* présente une parenté évolutive plus marquée pour l'enzyme de classe I (PTD de *L. helveticus*) et une appartenance fonctionnelle à la classe II, suggérant une divergence évolutive précoce dans l'évolution de ces enzymes. Son activité enzymatique pourrait se montrer différente de celles des autres espèces, et se prêter à la préparation d'un plus grand spectre de nucléosides.

## 5. CLONAGE PAR PCR INVERST DE QUATRE GENES NTD.

En utilisant des oligonucléotides dégénérés à partir de régions de la séquence en: acides aminés du polypeptide NTD de *L. leichmanii* (Hück, 1997) un fragment interne au gène ntd de *L. helveticus* a été amplifié. A partir de ce fragment, des oligonucléotides ont été synthétisés de façon à obtenir la totalité du gène par PCR inverse.

A partir des deux séquences ntd de *L. leichmannii* et de *L. helveticus*, nous avons redéfini des amorces consensus pour isoler les gènes ntd d'autres espèces de lactobacilles comme *L. acidophilus, L. crispatus, L. amylovorus* avec la même démarche que celle décrite ci-dessus.

## 6. Un crible nutritionnel pour distinguer les deux activités de désoxyribosyltransférases I et II.

Pour distinguer les deux activités désoxyribosyl transférase, l'ADN plasmidique de différentes colonies sélectionnées a été extrait puis utilisé pour transformer la souche PAK 26 auxotrophe pour la guanine et de la thymidine. Dans la souche PAK 26, le dTMP ne peut être synthétisé à partir du dUMP, parce que la thymidylate synthase codée par le gène thyA a été inactivé. De plus, la thymine ne peut être une source de thymidine parce que la thymidine phosphorylase codée par le gène deoA et l'uridine phosphorylase codée par le gène udp ont été délétés. La Déoxyguanosine (dR-G) et la thymine (T) seront les sources de guanine et de thymidine, seulement si une activité N-désoxyribosyl transférase II est exprimée dans la souche PAK 26 pour catalyser la réaction d'échange dG + T ⇔ dT + G. Seules les colonies exprimant une activité N-désoxyribosyl transférase II peuvent pousser sur un milieu glucose minéral supplémenté en déoxyguanosine et en thymine comme sources de guanine et de thymidine. Ce second criblage a par exemple permis de corréler l'activité N-désoxyribosyltransférase II (ntd) avec le plasmide pLH2 comprenant le polynucléotide de SEQ ID N°1 et codant pour l'enzyme ntd de *lactobacillus helveticus* et l'activité N-désoxyribosyltransférase I (ptd) avec le plasmide pLH4 comprenant le polynucléotide de SEQ ID N°3 et codant pour l'enzymz ptd de *lactobacillus helveticus*.

**Tableau 1: Croissance de la souche PAK 6 exprimant ou non une activité N-désoxyribosyltransférase sur milieu mineral glucose (*in vivo*) et activité enzymatique des extraits bruts correspondants (*in vitro*)**

| | *in vivo* | | | | *in vitro* | | |
|---|---|---|---|---|---|---|---|
| | A | G | dG | dG + A | dC + T | dG + A | dC + A |
| PAK 6 pSU19 | - | + | ± | - | - | - | - |
| PAK 6 Ll ntd | - | + | ± | + | + | + | + |
| PAK 6 Lh ntd | - | + | ± | + | + | + | + |
| PAK 6 Lh ptd | - | + | ± | + | - | + | - |
| PAK 6 Lf ntd | - | + | ± | + | + | + | + |

(+) croissance
(-) absence de croissance
PAK 6: MG1655 ΔguaBA::Apra, Δdeo
ntd L1: gène codant pour la N-désoxyribosyltransférase de *Lactobacillus leichmannii*
ntd Lh: gène codant pour la N-désoxyribosyltransférase de *Lactobacillus helveticus*
ntd Lf: gène codant pour la N-désoxyribosyltransférase de *Lactobacillus fermentum*
ptd Lh: gène codant pour la purine désoxyribosyltransférase de *Lactobacillus helveticus*
A: adénine; G: guanine; T: thymine; dG: désoxyguanosine; dC: désoxycytidine

## REFERENCES

Cadwell et al. (1992) Research 2 : 28-33.
Carson & Wasson (1988) Biochem. Biophys. Res. Comm. 155, 829-834.
Carson et al., (1990) Proc. Natl. Acad.Sci. USA 81, 2232-2236.
Carter (1993) Curr. Op. Biotechnology 3, 533.

Danzin & Cardinaud(1976) Eur. J. Biochem. 62, 365-372.
Duck et al. (1990), Biotechniques, 9, 142.
Epstein (1992) Médecine/Sciences, 8, 902.
Fischer et al., (1990) Ger. Offen. DE 3840160.
Guatelli et al. (1990), Proc. Natl. Acad. Sci. USA 87: 1874.
Isono (1988) J. Antibiotics 12, 1711-1739.
Kievitis et al., (1991) J. Virol. Methods, 35, 273.
Köhler et Milstein. (1975) Nature 256, 495.
Krenitsky et al., (1981) Biochemistry 20, 3615-3621.
Kwoh et al. (1989) Proc. Natl. Acad. Sci. USA, 86, 1173.
Landegren et al. (1988) Science 241, 1077.
Matthews et al. (1988) Anal. Biochem., 169, 1-25.
Miele et al., (1983) J. Mol. Biol., 171, 281-285.
Neddleman et Wunsch (1970) J. Mol, Biol. 48 : 443
Pearson et Lipman (1988) Proc. Natl. Acad. Sci. USA 85 : 2444
Périgaud et al., (1992) Annales de l'Institut Pasteur Actualités 3, 179-215.
Perricaudet et al. (1992), La Recherche 23 : 471.
Rolfs, A. *et al.* (1991), Berlin : Springer-Verlag.
Sambrook et al. (1989) Molecular cloning : a laboratory manual second edition - Cold Spring Harbor Laboratory Press. Cold Spring Harbor, NY. USA
Segev, (1992), Kessler C. Springer Verlag, Berlin, New-York, 197-205.
Smith et Waterman (1981) Ad. App. Math. 2 : 482
Temin, (1986) Retrovirus vectors for gene transfer. In Kucherlapati R., ed. Gene Transfer.
Walker et al. (1992) EMBO J. 1 : 945-951
Wilson et al. (1995) Synthesis 1465-1479.
Wong C.H. et al. (1995) Angew. Chem. Int. Ed. Engl. 34, 412-432 et 521-546.

1. Polypeptide isolé ou purifié de *Lactobacillus* ayant au moins une activité N-désoxyribosyltransférase de séquence d'acides aminés choisi parmi les séquences SEQ ID N°2, SEQ ID N°4, SEQ ID N°6, SEQ ID N°8, SEQ ID N°10, SEQ ID N°12.

2. Polypeptide isolé selon la revendication 1 **caractérisé en ce que** le polypeptide de SEQ ID N°2 est codé par la N-désoxyribosyltransférase codé par le gène ntd Lh de *Lactobacillus helveticus*.

3. Polypeptide isolé selon la revendication 1 **caractérisé en ce que** le polypeptide de SEQ ID N°4 est codé par la N-désoxyribosyltransférase codé par le gène ptd Lh de *Lactobacillus helveticus*.

4. Polypeptide isolé selon la revendication 1 **caractérisé en ce que** le polypeptide de SEQ ID N°6 est codé par la N-désoxyribosyltransférase codé par le gène ntd Lf de *Lactobacillus fermentum*.

5. Polypeptide isolé selon la revendication 1 **caractérisé en ce que** le polypeptide de SEQ ID N°8 est codé par la N-désoxyribosyltransférase codé par le gène ntd de *Lactobacillus crispatus*.

6. Polypeptide isolé selon la revendication 1 **caractérisé en ce que** le polypeptide de SEQ ID N°10 est codé par la N-désoxyribosyltransférase codé par le gène ntd de *Lactobaclllus amylovorus*.

7. Polypeptide isolé selon la revendication 1 **caractérisé en ce que** le polypeptide de SEQ ID N°12 est codé par la N-dèsoxyribosyltransférase codé par le gène ntd de *Lactobacillus acidophilus*.

8. Polypeptide isolé **caractérisé en ce qu**'il comprend un polypeptide choisi parmi :

a) un polypeptide de séquence SEQ ID N°2, SEQ ID N°4, SEQ ID N°6, SEQ ID N°8, SEQ ID N°10, SEQ ID N°12.
b) un polypeptide variant de polypeptide de séquences d'acides aminés défini en a) ;
c) un polypeptide homologue au polypeptide défini en a) ou b) et comportant au moins 80 % d'identité avec ledit polypeptide de a) ;
d) un fragment d'au moins 15 acides aminés consécutifs d'un polypeptide défini en a);
e) un fragment biologiquement actif d'un polypeptide défini en a), b) ou c).

9. Polypeptide selon les revendications 1 à 8 **caractérisé en ce qu'**il permet de satisfaire l'exigence en guanine de la souche PAK6 déposée à la CNCM le 2 mai 2001 sous le N° I-2664.

10. Polynucléotide purifié ou isolé **caractérisé en ce qu**'il code pour un polypeptide selon les revendications 1 à 9.

11. Polynucléotide selon la revendication 10 de séquence SEQ ID N°1, SEQ ID N°3, SEQ ID N°5, SEQ ID N°7, SEQ ID N°9, SEQ ID N°11, SEQ ID N°13.

12. Polynucléotide isolé **caractérisé en ce qu**'il comprend un polynucléotide choisi parmi :

a) SEQ ID N°1, SEQ ID N°3, SEQ ID N°5, SEQ ID N°7, SEQ ID N°9, SEQ ID N°11, SEQ ID N°13.
b) la séquence d'un fragment d'au moins 15 nucléotides consécutifs de la séquence SEQ ID N°1, SEQ ID N°3, SEQ ID N°5, SEQ ID N°7, SEQ ID N°9, SEQ ID N°11, SEQ ID N°13;
c) une séquence nucléique présentant un pourcentage d'identité d'au moins 70 %, après alignement optimal avec une séquence définie en a) ou b);
d) la séquence complémentaire ou la séquence d'ARN correspondant à une séquence telle que définie en a), b) ou c).

13. Polynucléotide selon les revendications 10 à 12 **caractérisé en ce que** son expression dans les souches PAK6 permet de satisfaire à l'exigence en guanine de la dite souche.

14. Utilisation d'un polynucléotide selon la revendication 12 en tant qu'amorce pour l'amplification ou la polymérisation de séquences nucléiques de N-désoxyribosyltransférases.

15. Utilisation d'un polynucléotide selon les revendications 10 à 13 en tant que sonde pour la détection de séquences nucléiques de N-désoxyribosyltransférases.

16. Vecteur recombinant de clonage et/ou d'expression comprenant un polynucléotide selon l'une des revendication 10 à 13 ou exprimant un polypeptide selon l'une quelconque des revendications 1 à 9.

17. Vecteur recombinant appelé pLH2 comprenant le polynucléotide SEQ ID N°1 tel que présent dans la souche bactérienne déposée à la CNCM le 30 mai 2001 sous le N°I-2676.

18. Vecteur recombinant appelé pLH4 comprenant le polynucléotide SEQ ID N°3 tel que présent dans la souche bactérienne déposée à la CNCM le 30 mai 2001 sous le N°I-2677.

19. Vecteur recombinant appelé pLF6 comprenant le polynucléotide SEQ ID N°5 tel que présent dans la souche bactérienne déposée à la CNCM le 30 mai 2001 sous le N°I-2678.

20. Vecteur recombinant appelé pLA comprenant le polynucléotide SEQ ID N°20 tel que présent dans la souche bactérienne déposée à la CNCM le 21 juin 2001 sous le N°I-2689.

21. Cellule hôte, **caractérisé en ce qu'**elle est transformée par un vecteur selon les revendications 16 à 20.

22. Bactérie transformée par le vecteur pLH2 comprenant le polynucléotide SEQ ID N°1 telle que déposée à la CNCM le 30 mai 2001 sous le N°I-2676.

23. Bactérie transformée par le vecteur pLH4 comprenant le polynucléotide SEQ ID N°3 telle que déposée à la CNCM le 30 mai 2001 sous le N°I-2677.

24. Bactérie transformée par le vecteur pLF6 comprenant le polynucléotide SEQ ID N°5 telle que déposée à la CNCM le 30 mai 2001 sous le N°I-2678.

25. Bactérie transformée par le vecteur pLA comprenant le polynucléotide SEQ ID N°9 telle que déposée à la CNCM le 21 juin 2001 sous le N°I-2689.

26. Organisme métazoaire animal ou végétal, excepté l'homme, **caractérisé en ce qu'**il comprend une cellule selon la revendication 21.

27. Procédé de préparation d'un polypeptide recombinant **caractérisé en ce que** l'on cultive une cellule hôte selon la revendication 21 ou une bactérie selon les revendications 22 à 25 dans des conditions permettant l'expression et éventuellement la sécrétion dudit polypeptide recombinant et que l'on récupère ledit polypeptide recombinant.

28. Polypeptide recombinant susceptible d'être obtenu par un procédé selon la revendication 27.

29. Anticorps monoclonal ou polyclonal et ses fragments caractérisés en ce qu'il lie sélectivement un polypeptide selon l'une des revendications 1 à 9 ou 28.

30. Procédé de synthèse enzymatique *in vitro* ou *in vivo* de désoxyribonucléotides **caractérisé en ce qu**'il comprend au moins une étape réactionnelle catalysée par une N-désoxyribosyltransférase selon l'une quelconque des revendications 1 à 9.

31. Procédé selon la revendication 30 **caractérisé en ce que** la dite N-désoxyribosyltransférase catalyse l'échange d'une première nucléobase présente dans un désoxyribonucléoside par une seconde nucléobase.

32. Procédé selon la revendication 31 **caractérisé en ce que** ladite seconde nucléobase est sélectionnée dans le groupe composé des purines liées par N9, des pyrimidines liées par N1, des azines liées par N1, des imidazoles liées par N1, lesdites secondes nucléobases pouvant porter des substitutions des hydrogènes aux positions non liées.

33. Procédé selon la revendication 32 **caractérisé en ce que** la dite seconde nucléobase est sélectionnée dans le groupe composé de la 6-méthyl purine, 2-amino-6-méthylmercaptopurine, 6-diméthylaminopurine, 5-azacytidine, 2,6-dichloropurine, 6-chloroguanine, 6-chloropurine, 6-aza-thymine, 5-fluoro-uracile, éthyl-4-amino-5-imidazole carboxylate, imidazole-4-carboxamide et 1,2,4-triazole-3-carboxamide.

34. Procédé selon la revendication 31 **caractérisé en ce que** la dite première nucléobase est sélectionnée dans le groupe composé de l'adénine, la guanine, la thymine, l'uracile et l'hypoxanthine.

35. Procédé *in vivo* selon les revendications 31 à 34 **caractérisé en ce qu**'il comprend en outre l'étape d'introduire dans la cellule hôte la première nucléobase présente dans un désoxyribonucléoside.

36. Procédé *in vivo* selon les revendications 31 à 34 **caractérisé en ce qu**'il comprend en outre l'étape d' introduire dans la cellules hôte la seconde nucléobase présente dans un désoxyribonucléoside.

37. Procédé *in vivo* selon les revendications 31 à 34 **caractérisé en ce qu**'il comprend en outre l'étape d'introduire dans la cellule hôte la première nucléobase présente dans un désoxyribonucléoside et la seconde nucléobase de manière simultanée et/ou décalée dans le temps.

## Revendications

1. Procédé de synthèse enzymatique *in vitro* de désoxyribonucléotides **caractérisé en ce qu'**il comprend au moins une étape réactionnelle catalysée par une N-désoxyribosyltransférase isolée ou purifiée, comprenant un polypeptide choisi parmi :

    a) un polypeptide de séquence SEQ ID No.4, SEQ ID No.6, SEQ ID No.8, SEQ ID No.10 ou SEQ ID No.12.
    b) un polypeptide comportant au moins 80 % d'identité avec SEQ ID No.4, SEQ ID No.6, SEQ ID No.8, SEQ ID No.10 ou SEQ ID No.12.

2. Procédé selon la revendication 1, **caractérisé en ce qu'**il comprend au moins une étape réactionnelle réalisée dans une cellule vivante isolée, cette étape réactionnelle étant catalysée par ladite N-désoxyribosyltransférase isolée.

3. Procédé selon la revendication 1 ou 2, **caractérisé en ce que** ladite N-désoxyribosyltransférase catalyse l'échange d'une première nucléobase présente dans un désoxyribonucléoside par une seconde nucléobase.

4. Procédé selon la revendication 3, **caractérisé en ce que** ladite seconde nucléobase est sélectionnée dans le groupe composé des purines liées par N9, des pyrimidines liées par N1, des azines liées par N1, des imidazoles liées par N1, lesdites secondes nucléobases pouvant porter des substitutions des hydrogènes aux positions non liées.

5. Procédé selon la revendication 4, **caractérisé en ce que** la dite seconde nucléobase est sélectionnée dans le groupe composé de la 6-méthyl purine, 2-amino-6-méthylmercaptopurine, 6-diméthylaminopurine, 5-azacytidine, 2,6-dichloropurine, 6-chloroguanine, 6-chloropurine, 6-aza-thymine, 5-fluoro-uracile, éthyl-4-amino-5-imidazole carboxylate, imidazole-4-carboxamide et 1,2,4-triazole-3-carboxamide.

6. Procédé selon la revendication 3, **caractérisé en ce que** la dite première nucléobase est sélectionnée dans le groupe composé de l'adénine, la guanine, la thymine, l'uracile et l'hypoxanthine.

7. Procédé selon l'une des revendications 3 à 6, **caractérisé en ce qu'**il comprend, en outre, l'étape d'introduire dans ladite cellule la première nucléobase présente dans un désoxyribonucléoside.

8. Procédé selon l'une des revendications 3 à 6, **caractérisé en ce qu'**il comprend en outre l'étape d'introduire dans ladite cellule la seconde nucléobase présente dans un désoxyribonucléoside.

**9.** Procédé selon l'une des revendications 3 à 6, **caractérisé en ce qu'**il comprend en outre l'étape d'introduire dans ladite cellule la première nucléobase présente dans un désoxyribonucléoside et la seconde nucléobase de manière simultanée et/ou décalée dans le temps.

**10.** Procédé selon l'une des revendications 1 à 9, **caractérisé en ce que** ladite N-désoxyribosyltransférase permet de satisfaire l'exigence en guanine de la souche PAK6 déposée à la CNCM le 2 mai 2001 sous le N° I-2664.

**11.** Polypeptide isolé ayant au moins une activité N-désoxyribosyltransférase, **caractérisé en ce qu'**il comprend un polypeptide choisi parmi :

a) un polypeptide de séquence SEQ ID No.4, SEQ ID No.6, SEQ ID No.8, SEQ ID No.10 ou SEQ ID No.12.
b) un polypeptide comportant au moins 80 % d'identité avec SEQ ID No.4, SEQ ID No.6, SEQ ID No.8, SEQ ID No.10 ou SEQ ID No.12.

**12.** Polypeptide isolé, **caractérisé en ce que** sa séquence consiste en SEQ ID No.4, SEQ ID No.6, SEQ ID No.8, SEQ ID No.10 ou SEQ ID No.12.

**13.** Polypeptide selon l'une des revendications 11 et 12, **caractérisé en ce qu'**il permet de satisfaire l'exigence en guanine de la souche PAK6 déposée à la CNCM le 2 mai 2001 sous le N° 1-2664.

**14.** Polynucléotide purifié ou isolé **caractérisé en ce qu'**il code pour un polypeptide tel que défini à l'une des revendications 11 à 12.

**15.** Polynucléotide isolé codant pour un polypeptide ayant une activité N-désoxyribosyltransférase, **caractérisé en ce qu'**il consiste en un polynucléotide choisi parmi :

a) un polynucléotide de séquence SEQ ID No.3, SEQ ID No.5, SEQ ID No.7, SEQ ID No.9 ou SEQ ID No.11.
b) un polypeptide comportant au moins 70 % d'identité avec SEQ ID No.3, SEQ ID No.5, SEQ ID No.7, SEQ ID No.9 ou SEQ ID No.11.

**16.** Polynucléotide selon l'une des revendications 11 à 15, **caractérisé en ce que** son expression dans la souche PAK6 déposée à la CNCM le 2 mai 2001 sous le N° I-2664 permet de satisfaire l'exigence en guanine de ladite souche.

**17.** Vecteur recombinant de clonage et/ou d'expression comprenant un polynucléotide tel que défini à l'une des revendications 26 à 29 ou exprimant un polypeptide tel que défini à l'une des revendications 22 à 25.

**18.** Cellule hôte, **caractérisée en ce qu'**elle est transformée par un vecteur selon la revendication 17.

**19.** Cellule hôte, **caractérisée en ce qu'**elle est transformée par l'un des vecteurs suivants :

- le vecteur pLH4 comprenant le polynucléotide SEQ ID N°3,
- le vecteur pLF6 comprenant le polynucléotide SEQ ID N°5, ou
- le vecteur pLA comprenant le polynucléotide SEQ ID N°9,

**20.** Cellule hôte, **caractérisée en ce qu'**elle est choisie parmi les suivantes :

- bactérie telle que déposée à la CNCM le 30 mai 2001 sous le N°I-2677 transformée à l'aide du vecteur pLH4 comprenant le polynucléotide SEQ ID N°3;
- bactérie telle que déposée à la CNCM le 30 mai 2001 sous le N°I-2678 transformée à l'aide du vecteur pLF6 comprenant le polynucléotide SEQ ID N°5 ;
- bactérie telle que déposée à la CNCM le 30 mai 2001 sous le N°I-2689 transformée à l'aide du vecteur pLA comprenant le polynucléotide SEQ ID N°9.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- DE 3840160

### Littérature non-brevet citée dans la description


- **de Man.** *J. Appl. Bacteriol.,* 1960, vol. 23, 130-135
- **Cadwell et al.** *Research,* 1992, vol. 2, 28-33
- **Carson ; Wasson.** *Biochem. Biophys. Res. Comm.,* 1988, vol. 155, 829-834
- **Carson et al.** *Proc. Natl. Acad.Sci. USA,* 1990, vol. 81, 2232-2236
- **Carter.** *Curr. Op. Biotechnology,* 1993, vol. 3, 533
- **Danzin ; Cardinaud.** *Eur. J. Biochem.,* 1976, vol. 62, 365-372
- **Duck et al.** *Biotechniques,* 1990, vol. 9, 142
- **Epstein.** *Médecine/Sciences,* 1992, vol. 8, 902
- **Guatelli et al.** *Proc. Natl. Acad. Sci. USA,* 1990, vol. 87, 1874
- **Isono.** *J. Antibiotics,* 1988, vol. 12, 1711-1739
- **Kievitis et al.** *J. Virol. Methods,* 1991, vol. 35, 273
- **Köhler ; Milstein.** *Nature,* 1975, vol. 256, 495
- **Krenitsky et al.** *Biochemistry,* 1981, vol. 20, 3615-3621
- **Kwoh et al.** *Proc. Natl. Acad. Sci. USA,* 1989, vol. 86, 1173
- **Landegren et al.** *Science,* 1988, vol. 241, 1077
- **Matthews et al.** *Anal. Biochem.,* 1988, vol. 169, 1-25
- **Miele et al.** *J. Mol. Biol.,* 1983, vol. 171, 281-285
- **Neddleman ; Wunsch.** *J. Mol, Biol.,* 1970, vol. 48, 443
- **Pearson ; Lipman.** *Proc. Natl. Acad. Sci. USA,* 1988, vol. 85, 2444
- **Périgaud et al.** *Annales de l'Institut Pasteur Actualités,* 1992, vol. 3, 179-215
- **Perricaudet et al.** *La Recherche,* 1992, vol. 23, 471
- **Sambrook et al.** Molecular cloning : a laboratory manual. Cold Spring Harbor Laboratory Press, 1989
- **Smith ; Waterman.** *Ad. App. Math.,* 1981, vol. 2, 482
- Retrovirus vectors for gene transfer. **Temin.** Gene Transfer. 1986
- **Walker et al.** *EMBO J.,* 1992, vol. 1, 945-951
- **Wilson et al.** *Synthesis,* 1995, 1465-1479
- **Wong C.H. et al.** *Angew. Chem. Int. Ed. Engl.,* 1995, vol. 34, 412-432521-546

# Listage de séquences

<110> INSTITUT PASTEUR

<120> N-désoxyribosyltransferases de lactobacilles, séquences nucléotidiques correspondantes et

leurs applications.

<130> BIF 023253 PCT

<140> PCT/FR02/03120
<141> 2002-09-12

<150> FR 0 111 911
<151> 2001-09-14

<160> 16

<170> PatentIn version 3.1

<210> 1
<211> 477
<212> DNA
<213> Lactobacillus helveticus NTD

<220>
<221> CDS
<222> (1)..(477)
<223>

<400> 1

```
atg aac aag aaa aag act tta tat ttt ggt gcc ggt tgg ttt aat gaa      48
Met Asn Lys Lys Lys Thr Leu Tyr Phe Gly Ala Gly Trp Phe Asn Glu
1               5                   10                  15

aag caa aac aaa gct tac aaa gaa gca atg gca gct tta aaa gaa aat      96
Lys Gln Asn Lys Ala Tyr Lys Glu Ala Met Ala Ala Leu Lys Glu Asn
            20                  25                  30

cca aca gtt gat tta gaa aat agt tat gtg ccc ctt gaa aac caa tac      144
Pro Thr Val Asp Leu Glu Asn Ser Tyr Val Pro Leu Glu Asn Gln Tyr
        35                  40                  45

aag ggt att cgc att gat gaa cat cca gaa tac ttg cac aac att gaa      192
Lys Gly Ile Arg Ile Asp Glu His Pro Glu Tyr Leu His Asn Ile Glu
    50                  55                  60

tgg gct tct gca acc tac cac aat gat tta gta gga att aag act tct      240
Trp Ala Ser Ala Thr Tyr His Asn Asp Leu Val Gly Ile Lys Thr Ser
65                  70                  75                  80

gat gtc atg ctt ggc gta tat ttg cca gaa gaa gaa gac gtc ggc tta      288
Asp Val Met Leu Gly Val Tyr Leu Pro Glu Glu Glu Asp Val Gly Leu
                85                  90                  95

ggc atg gaa ctg ggc tac gca tta tct caa gga aaa tat att tta ttg      336
Gly Met Glu Leu Gly Tyr Ala Leu Ser Gln Gly Lys Tyr Ile Leu Leu
            100                 105                 110

gtt atc cca gat gaa gat tac ggc aag cca atc aac tta atg agc tgg      384
Val Ile Pro Asp Glu Asp Tyr Gly Lys Pro Ile Asn Leu Met Ser Trp
        115                 120                 125

ggc gtt tgt gac aat gcc atc aag atc agt gaa tta aaa gac ttc gac      432
Gly Val Cys Asp Asn Ala Ile Lys Ile Ser Glu Leu Lys Asp Phe Asp
    130                 135                 140

ttt aac aag cct cgc tac aat ttc tac gac gga gct gta tat taa          477
Phe Asn Lys Pro Arg Tyr Asn Phe Tyr Asp Gly Ala Val Tyr
```

145 150 155

<210> 2
<211> 158
<212> PRT
<213> Lactobacillus helveticus NTD

<400> 2

```
Met Asn Lys Lys Lys Thr Leu Tyr Phe Gly Ala Gly Trp Phe Asn Glu
1               5                   10                  15

Lys Gln Asn Lys Ala Tyr Lys Glu Ala Met Ala Ala Leu Lys Glu Asn
            20                  25                  30

Pro Thr Val Asp Leu Glu Asn Ser Tyr Val Pro Leu Glu Asn Gln Tyr
        35                  40                  45

Lys Gly Ile Arg Ile Asp Glu His Pro Glu Tyr Leu His Asn Ile Glu
    50                  55                  60

Trp Ala Ser Ala Thr Tyr His Asn Asp Leu Val Gly Ile Lys Thr Ser
65                  70                  75                  80

Asp Val Met Leu Gly Val Tyr Leu Pro Glu Glu Glu Asp Val Gly Leu
                85                  90                  95

Gly Met Glu Leu Gly Tyr Ala Leu Ser Gln Gly Lys Tyr Ile Leu Leu
            100                 105                 110

Val Ile Pro Asp Glu Asp Tyr Gly Lys Pro Ile Asn Leu Met Ser Trp
        115                 120                 125

Gly Val Cys Asp Asn Ala Ile Lys Ile Ser Glu Leu Lys Asp Phe Asp
    130                 135                 140

Phe Asn Lys Pro Arg Tyr Asn Phe Tyr Asp Gly Ala Val Tyr
145                 150                 155
```

<210> 3
<211> 504
<212> DNA
<213> Lactobacillus helveticus PTD

<220>
<221> CDS
<222> (1)..(504)
<223>

<400> 3

```
atg aaa gca gta gtt cca aca gga aaa att tat tta ggc tca cca ttt       48
Met Lys Ala Val Val Pro Thr Gly Lys Ile Tyr Leu Gly Ser Pro Phe
1               5                   10                  15

tac agc gat gct caa aga gaa aga gca gct aag gca aaa gag ttg tta       96
Tyr Ser Asp Ala Gln Arg Glu Arg Ala Ala Lys Ala Lys Glu Leu Leu
            20                  25                  30

gca aaa aat cta agc atc gcg cac gtc ttc ttc ccc ttt gat gat ggt      144
Ala Lys Asn Leu Ser Ile Ala His Val Phe Phe Pro Phe Asp Asp Gly
```

```
            35                  40                  45

ttc acc gat cct gat gaa aag aat cct gaa att ggc ggc atc aga agc        192
Phe Thr Asp Pro Asp Glu Lys Asn Pro Glu Ile Gly Gly Ile Arg Ser
    50                  55                  60

atg gtt tgg cgg gat gca act tac caa aat gat tta act ggt att tcg        240
Met Val Trp Arg Asp Ala Thr Tyr Gln Asn Asp Leu Thr Gly Ile Ser
65                  70                  75                  80

aat gcc act tgt ggc gtc ttc tta tat gat atg gat caa tta gat gac        288
Asn Ala Thr Cys Gly Val Phe Leu Tyr Asp Met Asp Gln Leu Asp Asp
                85                  90                  95

ggc tct gcc ttt gaa att ggc ttc atg cgt gcg atg cat aag ccg gtg        336
Gly Ser Ala Phe Glu Ile Gly Phe Met Arg Ala Met His Lys Pro Val
            100                 105                 110

atc ttg gtg cca ttc act gag cat ccc gaa aaa gaa aag aaa atg aac        384
Ile Leu Val Pro Phe Thr Glu His Pro Glu Lys Glu Lys Lys Met Asn
        115                 120                 125

ctg atg atc gca caa ggc gta acc acc atc att gat ggc aat act gaa        432
Leu Met Ile Ala Gln Gly Val Thr Thr Ile Ile Asp Gly Asn Thr Glu
    130                 135                 140

ttt gaa aaa cta gct gat tat aac ttc aac gaa tgt cct ttt aat cca        480
Phe Glu Lys Leu Ala Asp Tyr Asn Phe Asn Glu Cys Pro Phe Asn Pro
145                 150                 155                 160

gtt cgc ggt tac ggt atc tat taa                                        504
Val Arg Gly Tyr Gly Ile Tyr
                165
```

<210> 4
<211> 167
<212> PRT
<213> Lactobacillus helveticus PTD

<400> 4

```
Met Lys Ala Val Val Pro Thr Gly Lys Ile Tyr Leu Gly Ser Pro Phe
1               5                   10                  15

Tyr Ser Asp Ala Gln Arg Glu Arg Ala Ala Lys Ala Lys Glu Leu Leu
            20                  25                  30

Ala Lys Asn Leu Ser Ile Ala His Val Phe Phe Pro Phe Asp Asp Gly
        35                  40                  45

Phe Thr Asp Pro Asp Glu Lys Asn Pro Glu Ile Gly Gly Ile Arg Ser
    50                  55                  60

Met Val Trp Arg Asp Ala Thr Tyr Gln Asn Asp Leu Thr Gly Ile Ser
65                  70                  75                  80

Asn Ala Thr Cys Gly Val Phe Leu Tyr Asp Met Asp Gln Leu Asp Asp
                85                  90                  95

Gly Ser Ala Phe Glu Ile Gly Phe Met Arg Ala Met His Lys Pro Val
            100                 105                 110

Ile Leu Val Pro Phe Thr Glu His Pro Glu Lys Glu Lys Lys Met Asn
```

```
            115                 120                 125

Leu Met Ile Ala Gln Gly Val Thr Thr Ile Ile Asp Gly Asn Thr Glu
    130                 135                 140

Phe Glu Lys Leu Ala Asp Tyr Asn Phe Asn Glu Cys Pro Phe Asn Pro
145                 150                 155                 160

Val Arg Gly Tyr Gly Ile Tyr
                165


<210>  5
<211>  516
<212>  DNA
<213>  Lactobacillus fermentum NTD

<220>
<221>  CDS
<222>  (1)..(504)
<223>

<400>  5
ttg aaa aat acc gac cca gtt gct aac act aaa att tac ctg gct acc      48
Leu Lys Asn Thr Asp Pro Val Ala Asn Thr Lys Ile Tyr Leu Ala Thr
1               5                   10                  15

agc ttc ttc aac gaa gaa caa cgt gcc cgc atc cct caa gct cta gcc      96
Ser Phe Phe Asn Glu Glu Gln Arg Ala Arg Ile Pro Gln Ala Leu Ala
            20                  25                  30

caa cta gaa gcc aac ccg act gtc ggc gtt gtt cac cag cca ttc gat     144
Gln Leu Glu Ala Asn Pro Thr Val Gly Val Val His Gln Pro Phe Asp
        35                  40                  45

ttc caa tat aaa gat gca cgc gta gac tcc gat cct gcc ggc gtc ttt     192
Phe Gln Tyr Lys Asp Ala Arg Val Asp Ser Asp Pro Ala Gly Val Phe
    50                  55                  60

ggc agc ctc gaa tgg caa att gcc act tac aat aac gac ctc aac gcg     240
Gly Ser Leu Glu Trp Gln Ile Ala Thr Tyr Asn Asn Asp Leu Asn Ala
65                  70                  75                  80

gta gga act tcc gat gtc tgc gtt gct tta tac gat atg gac caa att     288
Val Gly Thr Ser Asp Val Cys Val Ala Leu Tyr Asp Met Asp Gln Ile
                85                  90                  95

gac gaa gga att tgt atg gaa atc ggc atg ttc gtc gcc ctc cat aaa     336
Asp Glu Gly Ile Cys Met Glu Ile Gly Met Phe Val Ala Leu His Lys
            100                 105                 110

cct atc gtt tta cta cct ttt act aag aaa gat aag tct gct tat gaa     384
Pro Ile Val Leu Leu Pro Phe Thr Lys Lys Asp Lys Ser Ala Tyr Glu
        115                 120                 125

gct aac cta atg cta gca cgg ggt gta act acc tgg ttg gaa cct aat     432
Ala Asn Leu Met Leu Ala Arg Gly Val Thr Thr Trp Leu Glu Pro Asn
    130                 135                 140

gac ttt agt ccc tta aaa gac ttt aac ttt aac cac cca atg gct caa     480
Asp Phe Ser Pro Leu Lys Asp Phe Asn Phe Asn His Pro Met Ala Gln
145                 150                 155                 160

cct ttc cca cca ttc aag gtt ttc taactaacct aa                       516
Pro Phe Pro Pro Phe Lys Val Phe
                165
```

<210> 6
<211> 168
<212> PRT
<213> Lactobacillus fermentum NTD

<400> 6

Leu Lys Asn Thr Asp Pro Val Ala Asn Thr Lys Ile Tyr Leu Ala Thr
1               5                   10                  15

Ser Phe Phe Asn Glu Glu Gln Arg Ala Arg Ile Pro Gln Ala Leu Ala
            20                  25                  30

Gln Leu Glu Ala Asn Pro Thr Val Gly Val Val His Gln Pro Phe Asp
        35                  40                  45

Phe Gln Tyr Lys Asp Ala Arg Val Asp Ser Asp Pro Ala Gly Val Phe
    50                  55                  60

Gly Ser Leu Glu Trp Gln Ile Ala Thr Tyr Asn Asn Asp Leu Asn Ala
65                  70                  75                  80

Val Gly Thr Ser Asp Val Cys Val Ala Leu Tyr Asp Met Asp Gln Ile
                85                  90                  95

Asp Glu Gly Ile Cys Met Glu Ile Gly Met Phe Val Ala Leu His Lys
            100                 105                 110

Pro Ile Val Leu Leu Pro Phe Thr Lys Lys Asp Lys Ser Ala Tyr Glu
        115                 120                 125

Ala Asn Leu Met Leu Ala Arg Gly Val Thr Thr Trp Leu Glu Pro Asn
    130                 135                 140

Asp Phe Ser Pro Leu Lys Asp Phe Asn Phe Asn His Pro Met Ala Gln
145                 150                 155                 160

Pro Phe Pro Pro Phe Lys Val Phe
                165

<210> 7
<211> 255
<212> DNA
<213> Lactobacillus crispatus

<220>
<221> CDS
<222> (3)..(254)
<223>

<400> 7
ac aac cag tac aag ggt atc cgc gtt gat gaa cac cct gaa tac ttg      47
   Asn Gln Tyr Lys Gly Ile Arg Val Asp Glu His Pro Glu Tyr Leu
   1               5                   10                  15

cac gac att gaa tgg gca tca gct acc tac cat aac gac tta gta ggg      95
His Asp Ile Glu Trp Ala Ser Ala Thr Tyr His Asn Asp Leu Val Gly
                20                  25                  30

```
att aag tcc agc gac atc atg ctt ggc gtt tac ttg cct gaa gaa gaa      143
Ile Lys Ser Ser Asp Ile Met Leu Gly Val Tyr Leu Pro Glu Glu Glu
            35                  40                  45

gat gtt ggt ctg gga atg gaa ctt ggc tat gcc ctt tca aaa ggc aag      191
Asp Val Gly Leu Gly Met Glu Leu Gly Tyr Ala Leu Ser Lys Gly Lys
        50                  55                  60

tac atc ttg ttg gta att cct gat gaa gat tac ggt aag cca atc aac      239
Tyr Ile Leu Leu Val Ile Pro Asp Glu Asp Tyr Gly Lys Pro Ile Asn
    65                  70                  75

tta atg agc tgg ggc a                                                255
Leu Met Ser Trp Gly
80
```

<210> 8
<211> 84
<212> PRT
<213> Lactobacillus crispatus

<400> 8

```
Asn Gln Tyr Lys Gly Ile Arg Val Asp Glu His Pro Glu Tyr Leu His
1               5                   10                  15

Asp Ile Glu Trp Ala Ser Ala Thr Tyr His Asn Asp Leu Val Gly Ile
            20                  25                  30

Lys Ser Ser Asp Ile Met Leu Gly Val Tyr Leu Pro Glu Glu Glu Asp
        35                  40                  45

Val Gly Leu Gly Met Glu Leu Gly Tyr Ala Leu Ser Lys Gly Lys Tyr
    50                  55                  60

Ile Leu Leu Val Ile Pro Asp Glu Asp Tyr Gly Lys Pro Ile Asn Leu
65                  70                  75                  80

Met Ser Trp Gly
```

<210> 9
<211> 399
<212> DNA
<213> Lactobacillus amylovorus NTD

<220>
<221> CDS
<222> (1)..(399)
<223>

<400> 9

```
atg gaa gct tta aag aag aac cct act gtt gac tta gaa aac agt tac      48
Met Glu Ala Leu Lys Lys Asn Pro Thr Val Asp Leu Glu Asn Ser Tyr
1               5                   10                  15

gtc cca ctt gat aac caa tac aaa ggc atc cgc gtt gat gaa cac cca      96
Val Pro Leu Asp Asn Gln Tyr Lys Gly Ile Arg Val Asp Glu His Pro
            20                  25                  30

gaa tat tta cac gac att gaa tgg gca tca tct acc tac cac aat gac      144
Glu Tyr Leu His Asp Ile Glu Trp Ala Ser Ser Thr Tyr His Asn Asp
        35                  40                  45
```

```
tta gtt ggt att aag tct tca gac gta atg ctc ggt gtt tat tta cct       192
Leu Val Gly Ile Lys Ser Ser Asp Val Met Leu Gly Val Tyr Leu Pro
    50                  55                  60

gaa gaa gaa gat gtt ggc ctt ggg atg gaa ctt ggc tac gca ttg tct       240
Glu Glu Glu Asp Val Gly Leu Gly Met Glu Leu Gly Tyr Ala Leu Ser
65                  70                  75                  80

caa ggt aaa tac atc ttg ctt gtc atc cct gac gaa gac tat ggt aag       288
Gln Gly Lys Tyr Ile Leu Leu Val Ile Pro Asp Glu Asp Tyr Gly Lys
                85                  90                  95

cca atc aac ttg atg agc tgg ggc gtt tgc gac aac gta atc aag atc       336
Pro Ile Asn Leu Met Ser Trp Gly Val Cys Asp Asn Val Ile Lys Ile
            100                 105                 110

agc gaa ttg aaa gac ttc gac ttt aac aga cct cgc ttc aac ttt tac       384
Ser Glu Leu Lys Asp Phe Asp Phe Asn Arg Pro Arg Phe Asn Phe Tyr
        115                 120                 125

gat ggt gct gtc tat                                                   399
Asp Gly Ala Val Tyr
    130


<210>  10
<211>  133
<212>  PRT
<213>  Lactobacillus amylovorus NTD

<400>  10

Met Glu Ala Leu Lys Lys Asn Pro Thr Val Asp Leu Glu Asn Ser Tyr
1               5                   10                  15

Val Pro Leu Asp Asn Gln Tyr Lys Gly Ile Arg Val Asp Glu His Pro
            20                  25                  30

Glu Tyr Leu His Asp Ile Glu Trp Ala Ser Ser Thr Tyr His Asn Asp
        35                  40                  45

Leu Val Gly Ile Lys Ser Ser Asp Val Met Leu Gly Val Tyr Leu Pro
    50                  55                  60

Glu Glu Glu Asp Val Gly Leu Gly Met Glu Leu Gly Tyr Ala Leu Ser
65                  70                  75                  80

Gln Gly Lys Tyr Ile Leu Leu Val Ile Pro Asp Glu Asp Tyr Gly Lys
                85                  90                  95

Pro Ile Asn Leu Met Ser Trp Gly Val Cys Asp Asn Val Ile Lys Ile
            100                 105                 110

Ser Glu Leu Lys Asp Phe Asp Phe Asn Arg Pro Arg Phe Asn Phe Tyr
        115                 120                 125

Asp Gly Ala Val Tyr
    130


<210>  11
<211>  480
```

```
<212>  DNA
<213>  Lactobacillus acidophillus NTD

<220>
<221>  CDS
<222>  (1)..(480)
<223>

<400>  11
atg atg gca aaa aca aaa act tta tat ttc ggc gct ggt tgg ttt aat       48
Met Met Ala Lys Thr Lys Thr Leu Tyr Phe Gly Ala Gly Trp Phe Asn
1               5                   10                  15

gaa aag caa aat aag gct tat aaa gca gct atg gaa gct tta aaa caa       96
Glu Lys Gln Asn Lys Ala Tyr Lys Ala Ala Met Glu Ala Leu Lys Gln
            20                  25                  30

aac cct act gtt gat ttg gaa aat agt tat gtt cca ctt gaa aat caa      144
Asn Pro Thr Val Asp Leu Glu Asn Ser Tyr Val Pro Leu Glu Asn Gln
        35                  40                  45

tat aaa gat att cgt gtt gat gaa cat cct gaa tac tta cac gac att      192
Tyr Lys Asp Ile Arg Val Asp Glu His Pro Glu Tyr Leu His Asp Ile
    50                  55                  60

gaa tgg gca tct gct act tat cac aac gac tta att ggt atc aaa tct      240
Glu Trp Ala Ser Ala Thr Tyr His Asn Asp Leu Ile Gly Ile Lys Ser
65                  70                  75                  80

tca gat att atg tta ggg gtt tac tta cct gaa gaa gaa gat gtt ggt      288
Ser Asp Ile Met Leu Gly Val Tyr Leu Pro Glu Glu Glu Asp Val Gly
                85                  90                  95

ctt ggt atg gaa ctt ggc tac gca tta tca caa ggc aaa tat atc tta      336
Leu Gly Met Glu Leu Gly Tyr Ala Leu Ser Gln Gly Lys Tyr Ile Leu
            100                 105                 110

ctc gtt att cct gac gaa gat tat ggc aag cct atc aac ttg atg agt      384
Leu Val Ile Pro Asp Glu Asp Tyr Gly Lys Pro Ile Asn Leu Met Ser
        115                 120                 125

tgg ggt gta tgt gat aac gct att aag atc agc gaa ttg aag gac ttc      432
Trp Gly Val Cys Asp Asn Ala Ile Lys Ile Ser Glu Leu Lys Asp Phe
    130                 135                 140

gac ttc aat aag cca cgc ttt aac ttc tat gat ggc gct gta tat taa      480
Asp Phe Asn Lys Pro Arg Phe Asn Phe Tyr Asp Gly Ala Val Tyr
145                 150                 155


<210>  12
<211>  159
<212>  PRT
<213>  Lactobacillus acidophillus NTD

<400>  12

Met Met Ala Lys Thr Lys Thr Leu Tyr Phe Gly Ala Gly Trp Phe Asn
1               5                   10                  15


Glu Lys Gln Asn Lys Ala Tyr Lys Ala Ala Met Glu Ala Leu Lys Gln
            20                  25                  30


Asn Pro Thr Val Asp Leu Glu Asn Ser Tyr Val Pro Leu Glu Asn Gln
        35                  40                  45


Tyr Lys Asp Ile Arg Val Asp Glu His Pro Glu Tyr Leu His Asp Ile
    50                  55                  60
```

```
Glu Trp Ala Ser Ala Thr Tyr His Asn Asp Leu Ile Gly Ile Lys Ser
65                  70                  75                  80

Ser Asp Ile Met Leu Gly Val Tyr Leu Pro Glu Glu Glu Asp Val Gly
                85                  90                  95

Leu Gly Met Glu Leu Gly Tyr Ala Leu Ser Gln Gly Lys Tyr Ile Leu
            100                 105                 110

Leu Val Ile Pro Asp Glu Asp Tyr Gly Lys Pro Ile Asn Leu Met Ser
        115                 120                 125

Trp Gly Val Cys Asp Asn Ala Ile Lys Ile Ser Glu Leu Lys Asp Phe
    130                 135                 140

Asp Phe Asn Lys Pro Arg Phe Asn Phe Tyr Asp Gly Ala Val Tyr
145                 150                 155


<210>  13
<211>  795
<212>  DNA
<213>  Lactobacillus helveticus NTD

<220>
<221>  CDS
<222>  (140)..(616)
<223>

<220>
<221>  misc_feature
<222>  (133)..(133)
<223>  n means any nucleotide : a, g, c or t/u

<400>  13
aaaaaaattt tcagtattag tcattgaatt ttaccttcca ttatggaatt actattttta      60

gcgtaagtta acaagacgtt tttttcaatc gaaaatatgt taaagttaat tcgtcagcaa     120

tttttatggg ganaaaatt atg aac aag aaa aag act tta tat ttt ggt gcc      172
                     Met Asn Lys Lys Lys Thr Leu Tyr Phe Gly Ala
                     1               5                   10

ggt tgg ttt aat gaa aag caa aac aaa gct tac aaa gaa gca atg gca      220
Gly Trp Phe Asn Glu Lys Gln Asn Lys Ala Tyr Lys Glu Ala Met Ala
            15                  20                  25

gct tta aaa gaa aat cca aca gtt gat tta gaa aat agt tat gtg ccc      268
Ala Leu Lys Glu Asn Pro Thr Val Asp Leu Glu Asn Ser Tyr Val Pro
        30                  35                  40

ctt gaa aac caa tac aag ggt att cgc att gat gaa cat cca gaa tac      316
Leu Glu Asn Gln Tyr Lys Gly Ile Arg Ile Asp Glu His Pro Glu Tyr
    45                  50                  55

ttg cac aac att gaa tgg gct tct gca acc tac cac aat gat tta gta      364
Leu His Asn Ile Glu Trp Ala Ser Ala Thr Tyr His Asn Asp Leu Val
60                  65                  70                  75

gga att aag act tct gat gtc atg ctt ggc gta tat ttg cca gaa gaa      412
Gly Ile Lys Thr Ser Asp Val Met Leu Gly Val Tyr Leu Pro Glu Glu
                80                  85                  90
```

```
gaa gac gtc ggc tta ggc atg gaa ctg ggc tac gca tta tct caa gga        460
Glu Asp Val Gly Leu Gly Met Glu Leu Gly Tyr Ala Leu Ser Gln Gly
            95                  100                 105

aaa tat att tta ttg gtt atc cca gat gaa gat tac ggc aag cca atc        508
Lys Tyr Ile Leu Leu Val Ile Pro Asp Glu Asp Tyr Gly Lys Pro Ile
        110                 115                 120

aac tta atg agc tgg ggc gtt tgt gac aat gcc atc aag atc agt gaa        556
Asn Leu Met Ser Trp Gly Val Cys Asp Asn Ala Ile Lys Ile Ser Glu
    125                 130                 135

tta aaa gac ttc gac ttt aac aag cct cgc tac aat ttc tac gac gga        604
Leu Lys Asp Phe Asp Phe Asn Lys Pro Arg Tyr Asn Phe Tyr Asp Gly
140                 145                 150                 155

gct gta tat taa aaaataagca aactaaatga cctatcgctt aaaaattgcg           656
Ala Val Tyr

ataggtcatt ttttaatatt atctgtcatg tataaaatct ttcttaataa atatactcca     716

agtgattttc caaaaaaatt attattctat acccacttca tatggaagtc cgagtcactt     776

atgtaaatca tatatcact                                                  795
```

<210> 14
<211> 158
<212> PRT
<213> Lactobacillus helveticus NTD

<400> 14

```
Met Asn Lys Lys Lys Thr Leu Tyr Phe Gly Ala Gly Trp Phe Asn Glu
1               5                   10                  15

Lys Gln Asn Lys Ala Tyr Lys Glu Ala Met Ala Ala Leu Lys Glu Asn
            20                  25                  30

Pro Thr Val Asp Leu Glu Asn Ser Tyr Val Pro Leu Glu Asn Gln Tyr
        35                  40                  45

Lys Gly Ile Arg Ile Asp Glu His Pro Glu Tyr Leu His Asn Ile Glu
    50                  55                  60

Trp Ala Ser Ala Thr Tyr His Asn Asp Leu Val Gly Ile Lys Thr Ser
65                  70                  75                  80

Asp Val Met Leu Gly Val Tyr Leu Pro Glu Glu Glu Asp Val Gly Leu
                85                  90                  95

Gly Met Glu Leu Gly Tyr Ala Leu Ser Gln Gly Lys Tyr Ile Leu Leu
            100                 105                 110

Val Ile Pro Asp Glu Asp Tyr Gly Lys Pro Ile Asn Leu Met Ser Trp
        115                 120                 125

Gly Val Cys Asp Asn Ala Ile Lys Ile Ser Glu Leu Lys Asp Phe Asp
    130                 135                 140
```

```
Phe Asn Lys Pro Arg Tyr Asn Phe Tyr Asp Gly Ala Val Tyr
145                 150                 155


<210>  15
<211>  18
<212>  DNA
<213>  Lactobacillus leichmannii NTD1

<400>  15
agacgatcta cttcggtg                                                   18


<210>  16
<211>  18
<212>  DNA
<213>  Lactobacillus leichmannii NTD2

<400>  16
acggcacctt cgtagaag                                                   18
```